Europäisches Patentamt

European Patent Office

Office européen des brevets

(11) Publication number: **0 258 995 B1**

## EUROPEAN PATENT SPECIFICATION

(45) Date of publication of patent specification: **13.04.94**  (51) Int. Cl.5: **G01N 33/68**

(21) Application number: **87306613.8**

(22) Date of filing: **27.07.87**

(54) **Diagnosis, severity prediction, and monitoring of disease by immunoassay of free activation peptides of pancreatic zymogens.**

(30) Priority: **28.07.86 GB 8618333**
   **16.01.87 US 3728**

(43) Date of publication of application:
   **09.03.88 Bulletin 88/10**

(45) Publication of the grant of the patent:
   **13.04.94 Bulletin 94/15**

(84) Designated Contracting States:
   **AT BE CH DE ES FR GB GR IT LI LU NL SE**

(56) References cited:
   **WO-A-84/03564**
   **WO-A-84/04301**

   **P. KARLSON, "Kurzes Lehrbuch der Biochemie", 9th edition, 1974, chapter 8, Georg Thieme Verlag, Stuttgart (DE); p. 138&NUM;**

(73) Proprietor: **BIOSCIENCE INTERNATIONAL, INC.**
   **73 Rutland Street, Suite No. 1**
   **P.O. Box 18-1507**
   **Boston Massachusetts 02118(US)**

(72) Inventor: **Hermon-Taylor, John, Dept. of Surgery**
   **Cranmer Terrace,**
   **St. George's Hospital Med. School**
   **London SW17 ORE(GB)**
   Inventor: **Austen, Brian Maxwell, Dept. of Surgery**
   **Cranmer Terrace,**
   **St. George's Hospital Med. School**
   **London SW17 ORE(GB)**

(74) Representative: **Goldin, Douglas Michael et al**
   **J.A. KEMP & CO.**
   **14, South Square**
   **Gray's Inn**
   **London WC1R 5LX (GB)**

EP 0 258 995 B1

**Description**

Acute and chronic pancreatitis are human diseases which have shown a significant increase in frequency in recent years [1]. Acute pancreatitis presents as an abdominal emergency, whereas chronic pancreatitis in general involves the differential diagnosis of chronic or intermittent abdominal pain.

The exocrine pancreas produces and stores a range of digestive enzymes, including amylase and lipase, biosynthesised in active form, and others including trypsinogens, chymotrypsinogens, proelastases, procarboxypeptidases, and prophospholipases A2, synthesised as inactive proenzymes or zymogens. Zymogen activation normally occurs following secretion of pancreatic juice into the duodenal lumen, and involves the specific catalytic conversion of trypsinogen to active trypsin by duodenal enteropeptidase (E.C. 3.4.21.9). This removes from trypsinogen an amino-terminal oligopeptide containing the sequence tetra-L-aspartyl-L-lysine, an event followed by tryptic activation of the other proenzymes in a cascade of proteolytic cleavage. Activation peptides of pancreatic zymogens released in the course of this physiological process are thought to be degraded by duodenal oligopeptidases.

Acute pancreatitis is characterised by two distinct clinicopathological types [2]. In acute oedematous pancreatitis, pancreatic acinar cell damage is accompanied by leakage of inactive digestive zymogens with active amylase and lipase into the peritoneal cavity and circulation. The active enzymes are identifiable in the resulting ascitic fluid as well as in blood and urine, while proteinase digestive zymogens and prophospholipase remain unactivated. Although local inflammation and fat necroses due to active lipase occur, the condition is generally non-lethal and recovers within a few days of conservative management. In acute necrotising pancreatitis on the other hand, pancreatic acinar cell damage and digestive enzyme release is associated with varying degrees of digestive zymogen activation. Although complexs form between pancreatic proteinases and circulating macromolecular inhibitors, some of these complexs remain catalytically active. Phospholipase $A_2$ is also active in plasma. Widespread local and disseminated multiorgan damage results in a high morbidity and mortality.

The biochemical diagnosis of acute pancreatitis has traditionally relied on the detection in plasma, serum, urine, or ascitic fluid, of pancreatic digestive enzymes or their zymogens released from the pancreas itself as a result of the disease. The quantitation of total amylase activity in serum has been the principle diagnostic test employed [reviewed in ref. 3]. The arrival of sensitive immunological methods permitted the development of as says for pancreatic proteolytic enzymes and their zymogens including trypsin and trypsinogens [4-18], elastase [19,20], chymotrypsin [21], phospholipase [22,23], carboxypeptidases [24] and, in addition, lipase [25,26] and pancreatic trypsin inhibitor [27,28]. Some of these immunoassays particularly those using polyclonal antisera were complicated by the variable proteolytic degradation of their target molecules. Additional problems with these assays arise due to steric inhibition of antibody binding to target enzymes in complexs with macromolecular inhibitors [29,30]. Furthermore antibody recognising both parent zymogen and active enzyme is unable to distinguish between the two.

Amylase assays were improved to differentiate between pancreatic isoamylase and amylase activity contributed by other organs, particularly salivary glands [31-33]. Simultaneous detailed assessment of many of these tests in multiple patients with acute pancreatitis has, however, shown that the detection of lipase or immunoreactive trypsinogen does not improve initial diagnostic accuracy over the measurement of pancreatic isoamylase alone, and does not permit the biochemical recognition of the severe acute necrotising disease [34]. Amylase estimation in the diagnosis of pancreatic disease is complicated by sporadic cases of hyperamylasaemia, the need to distinguish pancreatic from other sources of amylase, the variable degradation of amylase activity in association with severe acute pancreatitis, and the inability to equate serum amylase levels with the severity of pancreatitis or to identify the onset of necrosis. Serum amylase estimations may also be unreliable in the recognition of ethanol-induced pancreatitis [35-37]. Attempts to improve the accuracy of severity prediction by assay for catalytically active pancreatic phospholipase A2 may prove a more fruitful approach [38]. This however is likely to have difficulties with specificity since phospholipases A2 are present in macrophages, granulocytes, platelets and other cells [39] and are elevated in blood in other acute abdominal conditions and septic shock [40].

The principal clinical diagnostic difficulty in acute pancreatitis therefore has been to distinguish severe abdominal pain with hyperamylasemia due to oedematous pancreatitis, from the more serious necrotising form in the earliest hours of the disease when intensive treatment, the use of specific enzyme inhibitors, and even surgical intervention and subtotal resection, may be life saving.

In chronic pancreatitis the diagnostic problem is different since subacute or intermittent abdominal pain due to chronic pancreatitis may not be associated with hyperamylasemia and distinction is required from the many other potential causes of such symptoms.

## THE INVENTION

The present invention is based upon the specific assay in body fluids for free pancreatic activation peptides PAP (the activation peptides of pancreatic zymogens specifically cleaved by limited proteolysis during activation) having available carboxy-termini. Such an assay will, for the first time, provide a precise method for recognising and quantifying pancreatic zymogen activation. Our invention is based on our appreciation that different forms of pancreatitis are associated with the specific release, or otherwise, of PAP. This general method will distinguish between forms of pancreatitis occurring with or without zymogen activation and will be clinically applicable to the diagnosis, severity prediction, and disease-course-monitoring in acute pancreatitis. It will also provide a precise diagnostic test for chronic pancreatitis in exacerbation.

In the test, a sample of the patient's body fluid particularly blood plasma, serum, urine or ascites will be assayed for one or more PAP. PAP available for such a strategy include the tetra-L-aspartyl-L-lysine ($D_4K$ in the single letter amino acid code used in relation to this and subsequently mentioned PAP) containing trypsinogen activation peptides (TAP) in which the $D_4K$ sequence is itself resistant to pancreatic proteolysis. Other suitable activation peptides are DSGISPR [41] the activation peptide of human pancreatic prophospholipase A2 (PLAP) and the APGPR [42] activation peptide of procolipase (CLAP). In addition proelastase I and II activation peptides [43] or derivatives of their limited proteolysis, as well as the large (90-100 amino acid) amino terminal moiety cleaved from procarboxypeptidases A and B, may also be considered. Of particular importance in this invention is the strategy of using antibody specifically carboxy-terminally directed against PAP. This will ensure that antibody binding and a positive result in the assay will only be reported when zymogen activation by limited proteolysis has indeed occurred, and that zymogens themselves in which PAP are linked by their carboxy-termini, will not be recognised.

## EXAMPLES

## IMMUNOASSAY OF TRYPSINOGEN ACTIVATION PEPTIDES (TAP)

In the following description, reference is frequently made to the immunoassay of trypsinogen activation peptides TAP. This is but one example of PAP and what is described in the following description in relation to TAP applies equally to the other PAP described above and below. When reference is made to TAP this is not to imply that the TAP that will be present will necessarily only be the pentapeptide $D_4K$ itself since additional amino terminal amino acid residues may be present. These differ among trypsinogen isoenzymes or may have been modified by amino terminal degradation. The TAP peptides recognised in the assay will include the $D_4K$ sequence at the carboxyterminus and it is the $D_4K$ sequence that is to be recognised.

The activation peptides of trypsinogens are highly conserved in vertebrate evolution and contain the polyanionic sequence $D_4K$ upstream of the lysyl-isoleucyl target bond that is cleaved during specific proteolytic activation [44,45]. The assay of the sample for the amino acid sequence $D_4K$ is most satisfactorily carried out using an antibody that recognises the $D_4K$ sequence with specificity for C-terminal $D_4K$ peptides and such antibodies form a further aspect of the present invention. These antibodies may be polyclonal or monoclonal. The polyclonal antibodies of the invention can be raised in animals by conventional techniques using as the immunogen $D_4K$ or $D_4K$ having a short leader sequence, e.g. alanyl-prolyl-phenylalanyl with or without cysteine at the amino terminus and either as free $D_4K$ containing peptide or $D_4K$ peptide haptenised prior to use as an immunogen. Where haptenisation is required, the $D_4K$ sequence or peptides including it can be chemically bonded to conventional peptide haptens such as bovine serum albumin (BSA) or thyroglobulin (TG).

When monoclonal antibodies are required, they may be prepared by conventional hybridoma technology, again using $D_4K$ or an amino acid sequence including $D_4K$ or $D_4K$ haptenised prior to use as the immunogen. Monoclonal antibodies to $D_4K$ may also be generated by using $D_4K$ bound to previously prepared anti-$D_4K$ antibody as the immunogen.

The present invention is based upon our appreciation that assay of PAP such as the $D_4K$ sequence can provide valuable unambiguous information concerning the existence and nature of pancreatic disorders in a patient. The exact way in which the assay is carried out is not critical and use may be made of any of the available direct or indirect (competitive) assays as well as two-site sandwich assays and assays involving quantification of antibody occupancy. The assay will normally involve the formation, in samples where $D_4K$ peptides are present, of a conjugate between the peptide including the $D_4K$ sequence and the antibody, said conjugate carrying a revealing label and being formed either in the solid phase or in the liquid phase of a solid/liquid phase reaction mixture, separating the solid phase from the liquid phase and determining the

3

presence of or amount of the revealing label in either the solid phase or the liquid phase as a measure of the presence of or amount of, respectively, peptide including the $D_4K$ sequence in the sample. Competitive assays normally require the attachment of the revealing label to competing $D_4K$, but labelling of a second anti-$D_4K$ antibody is necessary for the two-site sandwich assay. Any of the conventional revealing labels can be used, enzyme labels or radioactive labels being preferred although immunofluorescence can also be used. The selection of the enzyme or radioactive label may be $^{125}$I, one of the preferred radioactive labels, while horseradish peroxidase or alkaline phosphatase are the preferred enzymes. Biotin may also be used. Polyclonal or monoclonal anti-$D_4K$ carrying a revealing label form a further aspect of the present invention as does the pentapeptide $D_4K$ having a revealing label directly attached to one of its constituent amino acids and polypeptides including the labelled pentapeptide conjugated to another polypeptide. Amplification methods may be employed such as those which link alkaline phosphatase coupled to $D_4K$ peptide into the conversion of $NADP^+$ to $NAD^+$. The $NAD^+$ so formed then catalytically activates an $NAD^+$-specific redox cycle yielding an intensely coloured formazan dye [46].

The anti-$D_4K$ can be used, in accordance with the present invention, in conventional radio-immunoassays, in enzyme-linked or enzyme-multiplied immunoassays, in accordance with developed techniques, or in alternative immunoassays employing chemiluminescence, bioluminescence, photon emission fluorometry, electroluminescence, polarisation fluorescence, time resolved or other techniques. These would form the basis of reference laboratory immunoassays or simplified methods applicable to use in satellite laboratory, physicians office, emergency department, ward or intensive care unit. These methods frequently require the anti-$D_4K$ antibody or $D_4K$ itself to be immobilised by attachment to an inert solid substrate, such as latex particles or polystyrene balls or powder which can be packed into a column through which the test solutions can be run and such immobilised polyclonal or monoclonal anti-$D_4K$ and immobilised peptides comprising the $D_4K$ sequence having the lysine (K) as the carboxy terminus, with or without the attachment of a revealing label, form further aspects of the invention.

The invention also provides diagnostic test kits. Such kits comprise, as one component the labelled peptide of the invention (alone or conjugated to another peptide) or the labelled antibody of the invention. Alternatively, the test kit is one comprising a solid and a liquid component where the solid component is an immobilised peptide or antibody of the invention and one of the components is labelled, preferably with an enzyme or radioactive label.

In general, assays in accordance with the present invention using immobilised anti-$D_4K$ will include the step of bringing a liquid sample, suspected of including $D_4K$ peptides, into contact with a solid phase including a polyclonal or monoclonal anti-$D_4K$ in competition with $D_4K$ carrying a revealing agent, and assaying either the solid phase or the liquid phase for the presence of $D_4K$ carrying the revealing agent and taking the presence of that conjugate as a measure of the $D_4K$ content of the sample. Assays in accordance with the present invention will also include the method of bringing a liquid sample suspected of including $D_4K$ peptides, into contact with a solid phase with attached polyclonal or monoclonal anti-$D_4K$ ,separating the solid from the liquid phase, and quantifying the $D_4K$ bound by immobilised anti-$D_4K$, using a monoclonal second anti-$D_4K$ antibody carrying a revealing agent. An alternative method may also be used of quantifying antibody or immobilised $D_4K$ peptide occupancy in one or two dimensions on solid phase after exposure to the test sample, by the subsequent use of $D_4K$ peptides or antibody coupled to a revealing agent. Assays in accordance with the present invention will also include the method of adding anti-$D_4K$ polyclonal or monoclonal antibody with or without revealing agent, to the sample to be tested and bringing the treated sample into contact with $D_4K$ peptides immobilised on solid phase either directly or via an intermediate and such immobilised $D_4K$ peptide forms part of the present invention. $D_4K$ peptides on membranes or other solid phase support may be in the form of a disc which may have a central well, ring, or minicolumn. The test will be read by the intensity and/or distribution of anti-$D_4K$ binding to the immobilised $D_4K$ using the attached revealing agent or a revealing agent coupled to a second antibody to anti-$D_4K$ Ig. Assays in accordance with the present invention will also include the method of bringing the sample suspected of including $D_4K$ peptides into contact with a complex comprising anti-$D_4K$ antibody and $D_4K$, immobilised on an inert solid support and with or without a revealing agent. If the $D_4K$ is directly bound to the support, the antibody carries any revealing agent and vice versa. The displacement of initial $D_4K$ from the solid phase by $D_4K$ peptides in the sample may then be quantified.

The assay of the present invention may also be used to monitor the severity progress of pancreatitis. To do this, samples of body fluid are removed from the patient on at least two separate occasions spaced apart from one another by one to four hours and each sample is assayed for the concentration of $D_4K$ peptide. By taking several samples from the patient over a 24 hour period, it is possible to determine whether the severity of the patient's condition is changing and is such as to require surgery or other specific treatment.

The $D_4K$ peptides and other PAP are also of value in that they can be used for the purification, by affinity chromatography, of polyclonal or monoclonal anti-$D_4K$ or other anti-PAP. In such purification procedures, the $D_4K$ peptide or other PAP will normally be immobilised in a sterically accessible manner, on a solid support such as polystyrene or a polysaccharide such as a polydextran, a liquid sample containing relatively impure anti-$D_4K$ or other anti-PAP brought into contact with the immobilised $D_4K$ peptide or other PAP forming a $D_4K$/anti-$D_4K$ or other PAP/anti-PAP conjugate on the support. After washing the solid phase the anti-$D_4K$ or other anti-PAP is eluted to give a solution containing a relatively more pure form of anti-$D_4K$ or other anti-PAP.

The following Examples are given to illustrate the present invention.

In these Examples, single letter and three letter amino acid abbreviations are used as follows:

| D | Asp | Aspartic Acid |
|---|-----|---------------|
| K | Lys | Lysine |
| Y | Tyr | Tyrosine |
| C | Cys | Cysteine |
| A | Ala | Alanine |
| P | Pro | Proline |
| F | Phe | Phenylalanine |
| S | Ser | Serine |
| G | Gly | Glycine |
| I | Ile | Isoleucine |
| R | Arg | Arginine |
| T | Thr | Threonine |

SYNTHESIS OF ASP₄-LYS ($D_4K$) PEPTIDES

Peptides corresponding to the activation peptides found in human trypsinogens, Asp-Asp-Asp-Asp-Lys ($D_4K$), and Ala-Pro-Phe-Asp-Asp-Asp-Asp-Lys (APFD₄K), were synthesised with extra residues Tyr and Cys on their respective N-termini to provide side-chains that could be chemically cross-linked to protein carriers. Since the $D_4K$ sequence is degraded by aggressive acids, the base labile N-fluroeneylmethoxycarbonyl (Fmoc) group was used for reversible $\alpha$-N-terminal protection [47]. The peptides YD₄K and CAPFD₄K were assembled on polystyrene solid phase supports with the acid-labile p-alkoxybenzyl alcohol C-terminal linker [48]. A modification of the procedure of Meienhofer et al. [49] was used for the synthesis, omitting the dioxane:$H_2O$ wash which was found to be unnecessary, and deprotecting with 20% piperidine in DMF rather than dichloromethane.

Our initial studies showed that if one protected aspartyl group was coupled to the immobilised C-terminal (N-t-Boc)Lys residue, the dipeptide Asp-Lys cyclised to a diketopiperazine on base-catalysed removal of the $\alpha$-amino Fmoc group, and the moiety was released from the resin. The tetra-aspartyl sequence was therefore assembled using protected Asp-Asp dipeptides previously prepared, using hydroxybenzotriazole coupling to suppress racemisation during coupling to the resin-bound peptide. Completion of peptide bond formation after each addition was monitored with ninhydrin, or fluorescamine for Proline. Double couplings were required for each Asp-Asp dipeptide and for Fmoc(Trt)Cys. After simultaneous cleavage from the resin and deprotection of t-butyl side chain protecting groups with 50% TFA in $CH_2Cl_2$ - (containing also 5% ethyl methyl sulphide in the case of CAPFD₄K), peptides were purified by successive gel filtration and ion-exchange chromotography to yield the required peptides in 62% (YD₄K) and 35% (CAPFD₄K) yields. The peptides were shown to be homogenous as single ninhydrin-positive spots on high voltage paper electrophoresis at pH 6.5 ($R_{Asp}$YD₄K = 0.79, and $R_{Asp}$CAPFD₄K = 0.76), and satisfactory amino acid analysis after acid hydrolysis. We have previously shown by $^{13}$C-NMR that trypsinogen activation peptides synthesised by this procedure are free of $\alpha$-$\beta$ transpeptidation [50].

HAPTENISATION OF $D_4K$ PEPTIDES

Two haptens were prepared by specific amino terminal coupling of synthetic trypsinogen activation peptides to protein carriers. YD₄K was cross-linked to bovine serum albumin (BSA) by bis-diazotisation with the Tyr residue using benzidine dihydrochloride as described by Bassiri and Utiger (1972) [51]. Amino acid analysis of the purified adduct BSA-YD₄K showed a substitution of 8 peptides per BSA molecule. The sulphydryl group of the peptide CAPFD$_4$K was coupled to $\epsilon$-amino groups of Lys on bovine thyroglobulin (TG) using the heterobifunctional linker m-maleimidobenzoyl-N-hydroxysuccinimide [52] to yield TG-CAPFD$_4$K with a substitution of 40 molecules of peptide per thyroglobulin molecule.

## GENERATION OF SPECIFIC ANTI-D$_4$K ANTISERA

Emulsions of equal amounts of Freund's complete adjuvant (Miles Laboratories) with BSA-YD$_4$K 2.9 mg/ml or TG-CAPFD$_4$K 1.1 mg/ml were prepared by sonication on ice. NSW rabbits were immunised intradermally and intramuscularly with 2.0 ml of one or other emulsion and boosted using emulsions or corresponding hapten-peptide with Freund's incomplete adjuvant given each month. Sera were obtained before immunisation and 10 days after each challenge and tested for the presence of anti-D$_4$K antibodies using a solid-phase immunoradiometric assay. BSA-YD$_4$K and TG-CAPFD$_4$K conjugates were immobilised in separate 96-well polyvinylchloride microtitre plates (Dynatech No 1-220-24) by incubation of 50 $\mu$l at 60 $\mu$g/ml protein in 50mm Tris-HCl, 20mM CaCl$_2$, 0.1% sodium azide (TCA-buffer) at 4°C for 16h. Plates were then washed at room temperature with TCA-containing 10% (v/v) heat-irradiated horse serum (TCA-HS, Tissue Culture Services, Berkshire, UK), and excess sites blocked by incubation with TCA-HS for 1h. 50$\mu$l aliquots of immune sera to be tested were then added to the wells in the presence or absence of D$_4$K $10^{-5}$M and incubated for 2h at room temperature. Antisera from rabbits challenged with BSA-YD$_4$K was tested on immobilised TG-CAPFD$_4$K and vice-versa. Plates were then washed x3 with TCA-HS and incubated for a further 16h with 50$\mu$l $^{125}$I-goat antirabbit Ig (Miles AFA) 50,000 cpm per well in TCA-HS. Plates were washed, dried, and the wells counted in an LKB gamma counter. Titre of specific anti-D$_4$K antibody was obtained from the difference in cpm bound in the presence or absence of competing D$_4$K peptide $10^{-5}$M.

3 of 6 rabbits immunised with BSA-YD$_4$K developed anti-D$_4$K antibodies within 3 weeks. The titre in these animals increased over 2 to 3 months, then declined. In 2 of these animals the titre rose again with continued challenge to peak at 4 and again at 6 months. Three of the 4 rabbits immunised with TG-CAPFD$_4$K responded with anti-D$_4$K antibodies with peaks in the specific antibody titre at 2 and 3 and again at 6 months.

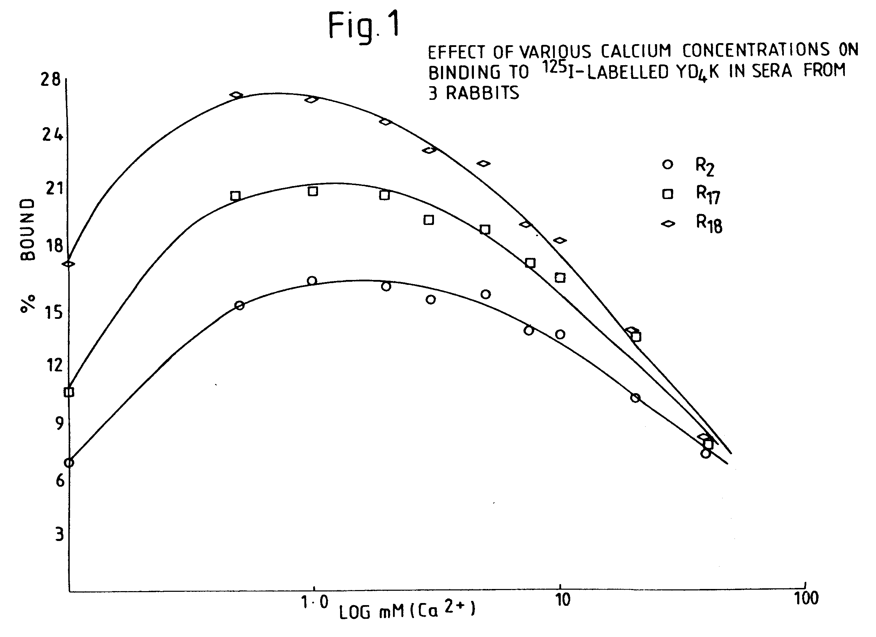

The presence of Ca$^{2+}$ion was found to enhance the binding of antibodies in immune sera to activation peptides, the extent of enhancement being different in individual rabbits. The sera from one rabbit (1) exhibited total dependance on Ca$^{2+}$ binding to $^{125}$I-YD$_4$K being reduced to background level in the presence of chelating amounts of EDTA. In 3 rabbits (Figure 1) binding increased in a concentration-dependant manner to a maximum observed at and above 1mM Ca$^{2+}$ and thereafter fell up to 40mM Ca$^{2+}$. Other divalent metal ions tested Mg$^{2+}$, Zn$^{2+}$, Ba$^{2+}$, Hg$^{2+}$ did not increase binding. The Ca$^{2+}$ dependency of subpopulations of anti-D$_4$K antibodies was thought to be the result of the Ca$^{2+}$ chelating properties of the two available pairs of Asp-$\beta$ carboxyls and the selective recognition of the peptide-Ca$^{2+}$chelate, rather than D$_4$K peptide alone, by rabbit host immunocytes following spontaneous Ca$^{2+}$binding in vivo.

### AFFINITY PURIFICATION OF ANTI-D$_4$K Ig FROM SPECIFIC ANTISERA

#### Preparation of affinity adsorbant

Activated CH-Sepharose 4B (Pharmacia Fine Chemicals) was suspended in 1mM HCl (100ml), and washed with a further 900ml of 1mM HCl. Then Tyr-Asp-Asp-Asp-Asp-Lys (60mg) in 25ml of 0.1M NaHCO$_3$ containing 0.5M NaCl was added and the gel shaken for 1h at 4°C. Excess ligand was washed away with 100ml of 0.1M NaHCO$_3$, and remaining active sites blocked by shaking the gel with 0.1M Tris-HCl (pH 8) at 4°C for 1h. The gel was then washed with 50ml 0.1M sodium acetate (pH 4) containing 0.5M NaCl. 50ml 0.1M Tris-HCl (pH 8), then these were washed twice more.

A small portion of the gel, hydrolysed in 6M HCl at 110°C for 20h, and subjected to amino acid analysis, showed that the extent of substitution was 1.10mmols/ml of gel.

#### Affinity chromatography on immobilised YD$_4$K

Two serum samples taken 6 weeks apart from rabbits, 1 and 2 (R$_1$, R$_2$) immunised with BSA-YD$_4$K, were pooled separately. Two similar samples from R17 and R18 immunised with TG-CAPFD$_4$K were pooled together. These pooled sera were subjected separately to affinity chromatography as follows: equal volumes of saturated solutions of ammonium sulphate were added to the sera, and the mixture left overnight at 4°C. The precipitated proteins were collected by centrifugation at 9,200 xgav at 4°C, and resuspended in the original volumes (see Table 1) of 50mM Tris-HCl (pH 7.4), 150mM NaCl, 20mM $CaCl_2$, and dialysed against two 2-litre changes of the same buffer. The dialysed protein was applied to a 1.5 x 10cm column of the Sepharose-immobilised $D_4K$ affinity matrix at a flow rate of 20 ml/h. Unbound protein was eluted with operational buffer until absorbance returned to zero. Then $Ca^{2+}$-dependent antibodies were displaced using 50mM Tris (pH 7.4), 150mM NaCl with 50mM EDTA followed by elution of $Ca^{2+}$-independent antibodies with 1M propionic acid. Fractions were dialysed, assayed for activity by binding $^{125}I$-$YD_4K$ (specific radioactivity $2.7 \times 10^8$ cpm/$\mu$g) and antibody containing fractions pooled.

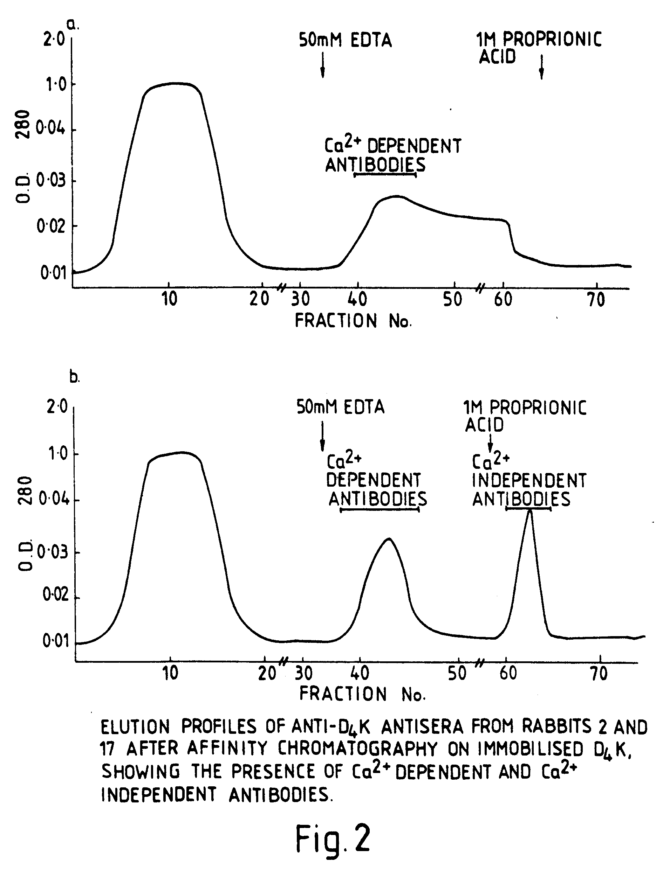

The elution profile of immunoglobulins from rabbit 1 showed that a wide peak of antibodies was obtained by elution with EDTA, but no protein or antibody eluted in 1M propionic acid (Fig.2a), confirming that anti-$D_4K$ antibodies present in this serum were $Ca^{2+}$-dependent. Table 1 shows that antisera from Rabbit 2 which had also been immunised with BSA-$YD_4K$ contained $Ca^{2+}$-dependent and $Ca^{2+}$-independent anti-$D_4K$ antibodies. This suggests the presence of 2 epitopes on the $D_4K$ molecule and that the recognition of one or both depended on the individual animal. The elution profile of pooled sera from rabbits 17 and 18 immunised with TG-$CAPFD_4K$ (Figure 2b) demonstrates both $Ca^{2+}$-dependent antibodies displaced with EDTA and $Ca^{2+}$-independent antibodies displaced using 1M propionic acid.

**TABLE 1:**

**AFFINITY PURIFICATION ON $YD_4K$ LINKED TO SEPHAROSE OF CALCIUM-DEPENDENT AND CALCIUM-INDEPENDENT ANTIBODIES**

**Rabbit 1 - Immunised with BSA-$YD_4K$:**

|  | Vol (ml) | Total Protein (mg) | Total Activity | Specific Activity | Yield | Purification |
|---|---|---|---|---|---|---|
| Pooled serum | 5 | 500 | 175[a] | 0.35 | 100 | 1 |
| EDTA-peak | 5 | 0.63 | 105[a] | 66 | 60 | 180 |
| Proprionic acid peak | 8 | 0 | 0[a] | 0 | 0 | - |

**Rabbit 2 - Immunised with BSA-$YD_4K$:**

|  | Vol (ml) | Total Protein (mg) | Total Activity | Specific Activity | Yield | Purification |
|---|---|---|---|---|---|---|
| Pooled serum | 16 | 1092 | 286[b] | 0.26 | 100 | 1 |
| EDTA-peak | 11 | 3.3 | 48[b] | 14.3 | 16.7 | 55 |
| Proprionic acid peak | 37 | 9.0 | 152[b] | 17.0 | 53.2 | 65 |

**Rabbits 17/18 - Immunised with TG-$CAPFD_4K$:**

|  | Vol (ml) | Total Protein (mg) | Total Activity | Specific Activity | Yield | Purification |
|---|---|---|---|---|---|---|
| Pooled serum | 21 | 1848 | 709[b] | 0.38 | 100 | 1 |
| EDTA-peak | 52 | 9.5 | 90[b] | 9.47 | 12.7 | 74 |
| Proprionic acid peak | 44 | 10.5 | 613[b] | 58.3 | 86.4 | 174 |

(a) % binding to $^{125}I$-$YD_4K$ at 1:1,000 dilution X volume (ml)

(b) difference in cpm in presence of $10^{-5}$ M $D_4K$ and absence of $D_4K$ binding to immobilised peptide at 1:100 dilution; detection by $^{125}I$-goat anti-rabbit X volume (ml)

## SPECIFICITY OF ANTIBODIES FOR C-TERMINAL $D_4K$ PEPTIDES

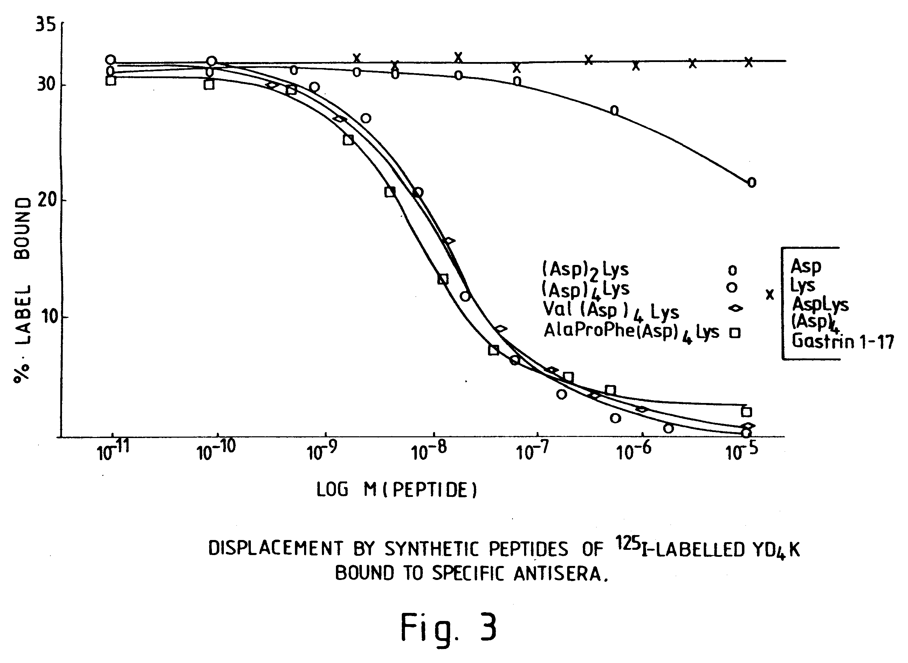

The relative affinities of affinity-purified $Ca^{2+}$-dependent antibodies present in sera from Rabbit 1 for various peptides was examined by determining competition of binding to $^{125}I$-$YD_4K$, measured by precipitation with a second antibody, donkey anti-rabbit. Synthetic peptides $D_4K$, $VD_4K$ and $APFD_4K$, displaced binding at similar concentrations (Figure 3) with $IC_{50}$ values all in the region of $10^{-8}$ M. In contrast Asp, Lys, Asp-Lys, Asp-Glu, poly-Asp, and human gastrin (residues 1 to 17 containing a pentaGlu sequence) did not displace binding to $^{125}I$-$YD_4K$ at concentrations as high as $10^{-4}$ M (Figure 3). Some binding to $Asp_2Lys$ was seen. Affinity purified $Ca^{2+}$-dependent and $Ca^{2+}$-independent antibodies from other rabbits showed a very similar specific affinity and specificity for tetra-L-aspartyl-L-lysine sequences.

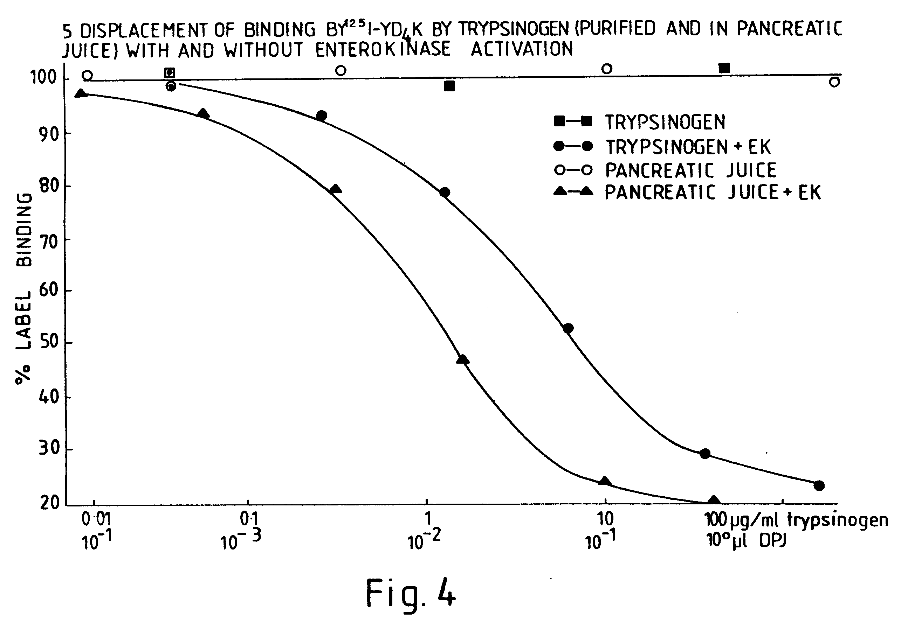

Of great importance to the general principle of the PAP assay of which this is an example, and for the application of these specific anti-$D_4$K antibodies to the diagnosis of pancreatic disease was the finding that anti-$D_4$K antibodies were C-terminally directed on the peptide, and were not displaced by trypsinogen (where the peptide is C-terminally bound to the protein) in concentrations of the zymogen of up to 100$\mu$g/ml. Upon incubation of trypsinogen with enteropeptidase, however, released $D_4$K-containing activation peptides actively displaced the binding of synthetic $^{125}$I-YD$_4$K giving 50% displacement after activation of a 2$\mu$g/ml solution of trypsinogen (Figure 4). Trypsinogen in native dog pancreatic juice was also activated by enteropeptidase (Figure 4) releasing immunoreactive $D_4$K peptides, giving 50% displacement in competitive solution-phase immunoassay after activation of $10^{-8}$L pancreatic juice. The novel $D_4$K-specific antibodies described here therefore also provide a new sensitive assay for enteropeptidase and trypsinogen applicable to fluids with endogenous trypsin inhibitors.

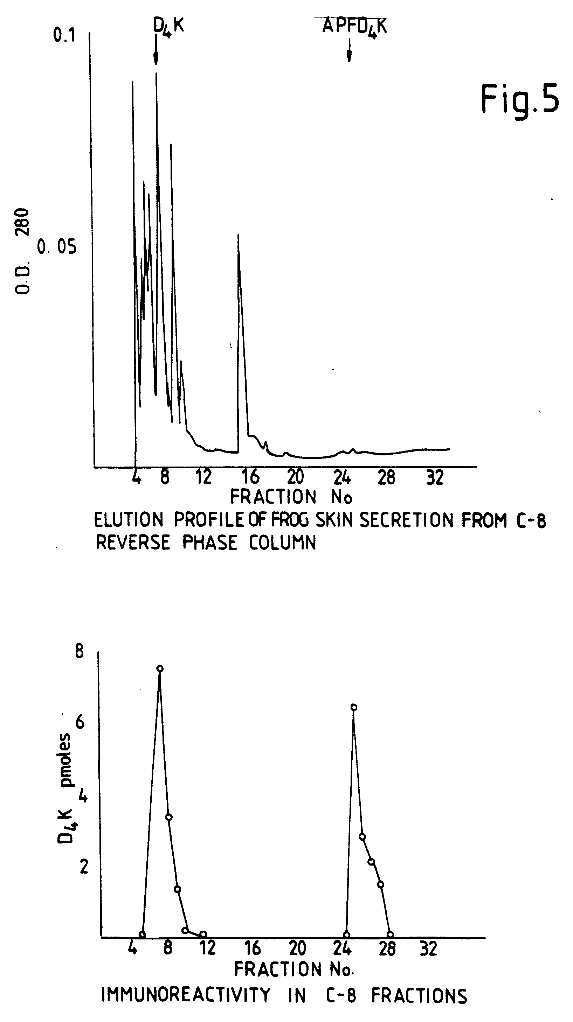

The singular specificity for $D_4$K peptides of the novel anti-$D_4$K antibodies we produced, was further demonstrated by examining skin secretions of the amphibian Xenopus laevis. Tetra-aspartyl-lysyl peptides as internal sequences in larger proteins, had been predicted to occur in the skin of this species by finding the corresponding nucleic acid sequence in cDNA clones derived from cutaneous mRNA [53]. Skin secretions from this amphibian are known to contain a variety of peptides with counterparts in the mammalian nervous system including CCK, gastrin, enkephalin, TRH, and somatostatin. We obtained a crude peptide extract derived from the skin of this species and subjected it to reverse-phase HPLC on a C8 column. The elution profile demonstrating multiple peptides is shown in Figure 5. Only two peaks immunoreactive for $D_4$K peptides were, however, identified and these co-chromatographed with the migration positions identified in this system for synthetic $D_4$K and APFD$_4$K. Free $D_4$K C-termini were thought to have been generated in the crude peptide mixture by processing of precursors, although the titre of available $D_4$K C-termini was increased by tryptic hydrolysis of the peptide extract indicating the presence of some unprocessed precursor. $D_4$K sequences in frog skin are thought to arise as a result of distant evolutionary relationships to mammalian trypsinogens. The specific recognition of $D_4$K peptide in the crude mixture obtained from this amphibian, further demonstrates the specificity of the affinity-purified anti-$D_4$K antibody.

## IMMUNOASSAY OF $D_4$K PEPTIDES

### Solution phase competitive immunoradiometric assay

100$\mu$l of a 1:250 dilution of anti-$D_4$K antiserum or a dilution of affinity-purified anti-$D_4$K antibodies in 50mM Tris-HCl, 20mM CaCl$_2$, 0.1% (w/v) BSA, pH 7.4 (RIA buffer) were added to 100$\mu$l of $^{125}$I-YD$_4$K, (10,000 cpm) in RIA buffer containing 0.2% (v/v) normal rabbit serum in polystyrene tubes. Then 100$\mu$l of solutions containing various concentrations of standard peptides or unknown plasma samples diluted in RIA-buffer were added, followed by 50$\mu$l of donkey anti-rabbit Ig serum (Wellcome Biotechnology) diluted 1:10 with RIA-buffer with 0.2% normal rabbit serum. The mixtures were incubated 18h at 4°C, then the tubes were centrifuged at 3,000rpm for 45min at 4°C, and the supernatants aspirated. The radioactivity in the precipitates was determined in an LKB rack-gamma counter.

### Solid-phase competitive immunoradiometric assay

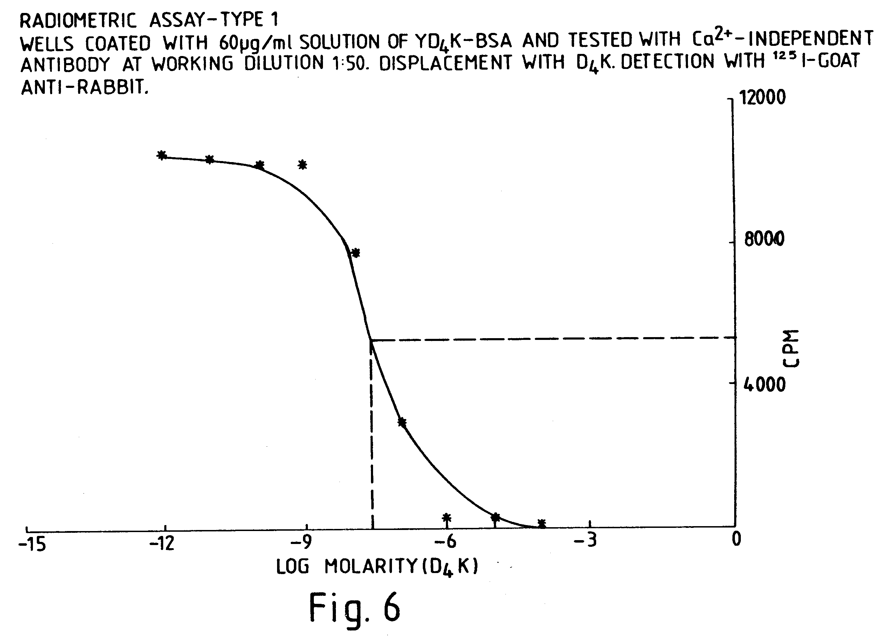

In order to improve the ease and sensitivity of immunoassay over that associated with competitive precipitation radioimmunoassay, two types of assay were developed using solid-phase techniques. In the first type, wells of PVC microtitre plates were first coated with BSA-YD$_4$K at 60$\mu$g/ml protein concentration. Then standard concentrations of synthetic activation peptides were preincubated with affinity-purified anti-$D_4$K antibodies and applied to the wells. Antibody adhering to the immobilised peptide hapten was then determined with $^{125}$I-labelled goat anti-rabbit antibody. A typical standard curve is shown in Figure 6, using Ca$^{2+}$-independent antibody. A steep dependence on the concentration of competing peptide was apparent over the range $10^{-6}$M to $10^{-9}$M with IC$_{50}$ value of 5 x $10^{-7}$

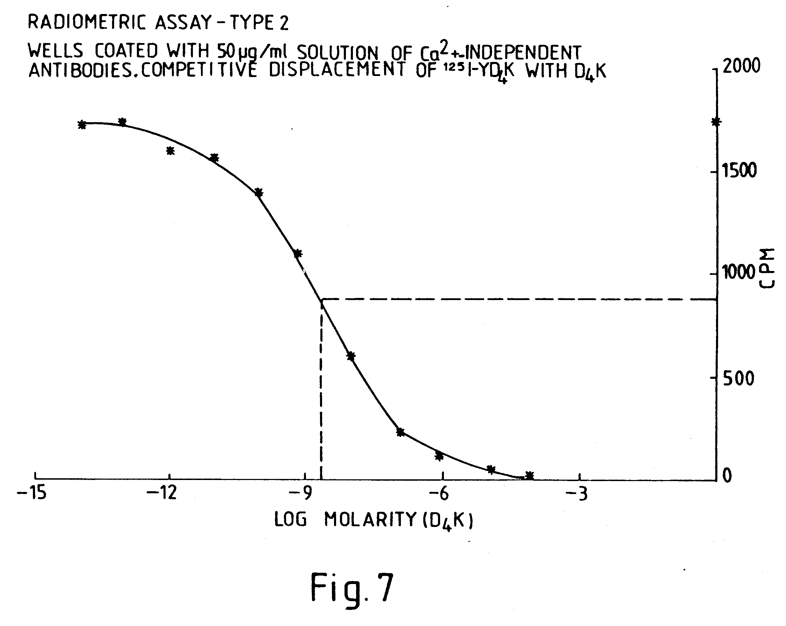

A second type of radiometric assay was developed using immobilised affinity-purified anti-$D_4$K antibody. Wells of microtitre plates were coated with 50$\mu$l of Ca$^{2+}$ independent antibodies (50$\mu$g/ml) at 4°C overnight, in 50mM Tris-HCl (pH 7.4) with 0.1% (w/v) sodium azide. Plates were then washed three times with 50mM Tris-HCl (pH 7.4) containing 10% horse-serum (T-HS buffer), and incubated with T-HS buffer for 1h at room temperature to block remaining sites. Solutions (60$\mu$l) were prepared containing $^{125}$I-YD$_4$K (100,000 cpm) and various concentrations of $D_4$K ($10^{-4}$M -$10^{-13}$M), in T-HS buffer. 50$\mu$l aliquots were transferred to the antibody-coated wells, then incubated at room temperature for 5h. Plates were washed

8

with T-HS buffer three times, and left to dry at room temperature. The wells were cut out and radioactivity determined as described. The results (Figure 7) showed a wider dependence on cpm bound between $10^{-6}$M and $10^{-12}$M of competing peptide, and an $IC_{50}$ value of about $10^{-9}$M, representing a 10-fold increase in sensitivity.

## Solid-phase enzyme-linked immunoassay

Benzidine dihydrochloride (10.2mg) in 0.2M HCl (2ml) was diazotised by addition of sodium nitrite (7.8mg) in water (0.2ml) for 1h at 4°C, then buffered to pH 9 with 3.2ml of 0.25M sodium borate/0.2M NaCl. Then $YD_4K$ (4.9mg) was coupled to horse-radish peroxidase (Sigma; 49.2mg) in 10.8 ml 0.16M sodium borate/0.14M NaCl (pH 9) by addition of the benzidine reagent at $+4°C$, and incubating for 2h. Excess reagents and peptide were removed by extensive dialysis against 0.15M NaCl, water, then 0.15M NaCl to yield a solution of conjugate of 3.2 mg/ml.

ELISA assay was performed as described above for the second type of radiometric assay using plates coated with $Ca^{2+}$ independent antibody (25μg/ml) in 50mM Tris (pH 7.4), 0.1% sodium azide. Plates were washed three times with 50mM Tris (pH 7.4), 10% (v/v) horse serum, 0.5% Tween 20, blocked with 50mM Tris (pH 7.4), 10% (v/v) horse serum for 30min, then incubated with a mixture of 1:10,000 peroxidase-$D_4K$ peptide conjugate and unknown samples or standard amounts fo peptide in 50mM Tris (pH 7.4), 0.05% Tween® 20. After 1h at 20°C, the plates were washed three times and developed by incubation with 3,3',5,5'- tetramethylenebenzidine (0.01% [w/v] in 0.1M sodium acetate/citric acid [pH 6.6]) for 1h at 20°C. The reaction was stopped by addition of 50ml 2M $H_2SO_4$, and colour estimated at 450nm. The resulting displacement curve exhibited similar sensitivity to the radiometric assay shown in Figure 7.

## ASSAY OF THE STABILITY AND DISTRIBUTION OF $D_4K$ PEPTIDES IN VIVO

The $D_4K$ peptide was found to be stable in human serum, and activated pancreatic juice. Immunoreactive $D_4K$ was unaltered by incubation with undenatured human serum at 37°C for 6h and 4°C for 48h .Sephadex G-100 chromatography of $YD_4K$ after incubation in human serum showed that the immunoreactive $D_4K$ peptide co-chromatographed with free peptide in buffer and did not complex with a serum component.

The stability of $APFD_4K$ and $D_4K$ were examined by incubation with activated pancreatic juice, and the chemical identity of the product examined by high-voltage paper electrophoresis at pH 6.5. The results showed that $APFD_4K$ was rapidly converted to free Ala, Pro and Phe and $D_4K$, but that the $D_4K$ sequence itself was stable for 24h at 37°C. In three separate experiments, duplicate 65 to 75 mg portions of fresh dog pancreas were placed in modified Bank's medium and incubated in the presence or absence of 100 units enterokinase (Sigma Chem.Co. E0885) at 37° for 4 hours and 22°C for a further 20 hours. Duplicate 100 μl samples of culture medium supernatant were taken at intervals, diluted 2:1 with RIA buffer, boiled for 10 mins and centrifuged. Supernatants were then stored at -20°C prior to assay for $D_4K$ peptides.

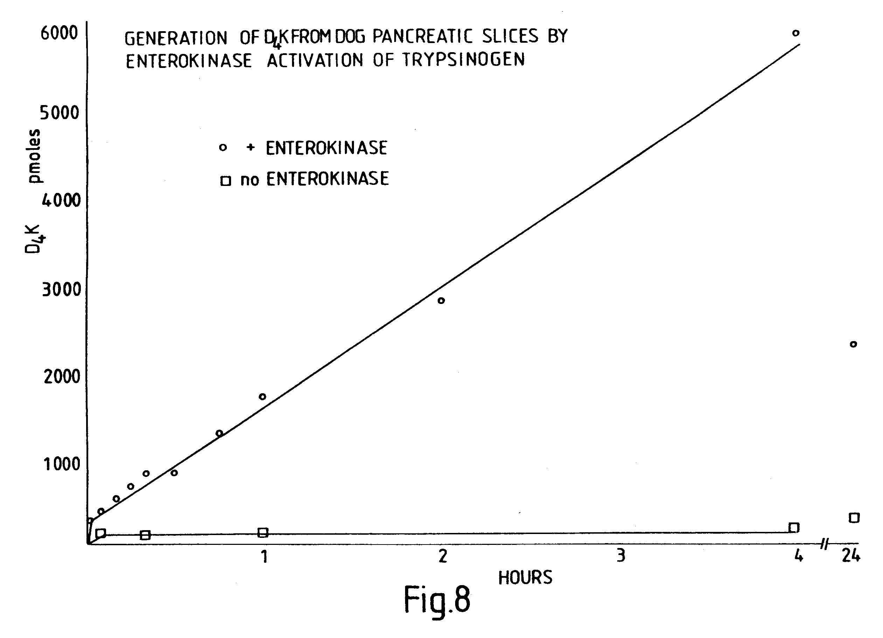

In each of the three experiments incubation with enterokinase was associated with degradation of pancreatic tissue and rapid release of immunoreactive $D_4K$ peptides. These changes were not seen in short term cultures without enterokinase (Figure 8). $D_4K$ immunoreactive peptides in the culture medium persisted at high levels after 24 hours incubation demonstrating their resistance to combined pancreatic proteolysis.

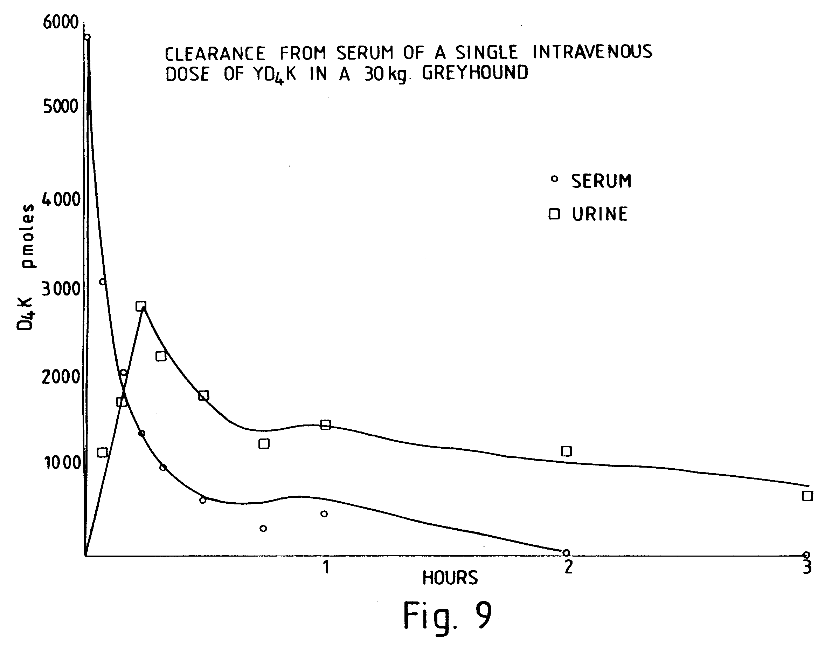

To determine the fate of circulating peptide, $D_4K$ (0.28ml, 1 mg/ml) was injected into the left femoral vein of rats under phenobarbital anaesthesia. Blood samples (0.5ml) were collected every 15min for 2h, and subjected to immunoassay. The results showed that administered immunoreactive $D_4K$ disappeared from serum in vivo with half-life of about 15min. In a second series of such experiments $YD_4K$ 100μg/kg in 1 ml PBS was injected intravenously in each of 3 healthy fasted greyhound dogs. Samples of blood and urine were obtained at intervals over 4 hours and stored at -20°C prior to assay. The mean half-life of $D_4K$ peptide in blood was 8.3 mins in dogs but in each case $D_4K$ was detectable in serum for 2 hours. $D_4K$ appeared in urine within 5 minutes of intravenous administration and persisted longer, for 3 to 4 hours.- (Figure 9)

A limited study was carried out to determine the tissue distribution of immunoreactive $D_4K$ peptides. Extracts of rat brain, pancreas, duodenum and pituitary were prepared by homogenisation and sonication in 24mM HCl containing pepstatin 0.1mg/ml, chymostatin 0.1 mg/ml, elastinal 0.1 mg/ml, and Trasylol 2000 KN/ml. Extracts were adjusted to pH 5.6, diluted with 0.1M succinate buffer pH 5.6, and digested with enteropeptidase for 1h at 37°C. 6 x $10^{-7}$ moles $D_4K$/mg protein and 1.5 x $10^{-7}$ moles $D_4K$/mg protein were found after enteropeptidase digestion in extracts of rat pancreas and duodenum respectively. No $D_4K$

peptide was detected in extracts of rat brain or pituitary, and none was found after assay of extracts of guinea-pig brain similarly prepared. Canine pancreatic juice obtained by cannulation of the main pancreatic duct followed by CCK stimulation, showed no immunoreactive $D_4K$ peptide before enteropeptidase activation and 5.7 x $10^{-4}$ moles $D_4K$/ml after activation. No $D_4K$ immunoreactivity was identified in fasting or postprandial serum or urine in multiple samples from 3 dogs and 6 normal human subjects. No $D_4K$ immunoreactivity was found in serum samples of patients with perforated duodenal ulcer, mesenteric infarction, appendicitis, aortic aneurysm, Crohn's disease or gastrointestinal haemorrhage. No $D_4K$ was found in human sera in association with Altzheimer's disease, rheumatoid arthritis or any other non pancreatic disease state tested.

## ASSAY OF $D_4K$ PEPTIDE IN EXPERIMENTAL AND HUMAN PANCREATITIS

Acute necrotising pancreatitis was induced experimentally in 22 anaesthetised rats by the controlled intrapancreatic duct microinfusion over 30 minutes of 75 $\mu$l buffer containing 10-20 mmol/l glycodeoxycholate alone or with 50ng highly-purified human enteropeptidase [54]. The rats subsequently received continuous intravenous analgesia together with pancreatic stimulation using CCK-33 10 IDU/kg/h. Blood was taken before the intraduct infusion and 3 hours afterwards, and assayed for free $D_4K$ peptide. Histological examination of the pancreas showed severe acute pancreatitis in every case. Circulating immunoreactive $D_4K$ peptide rose with the development of the disease in each of the 22 animals from an apparent mean basal level of 5 pmole/ml to a mean of 84 pmole/ml after 3 hours. By contrast no free $D_4K$ peptides were identified in pancreas homogenates from 6 mice with acute oedematous pancreatitis induced by CCK-8 hyperstimulation [55].

Preliminary clinical studies were carried out to determine whether $D_4K$ peptide could be identified in serum or urine from severe acute pancreatitis patients as predicted. Random serum and in some cases urine samples were accumulated from 22 patients with pancreatitis. Not all of these samples were taken during the early hours of the disease. On the basis of Ranson's criteria [56] 14 patients were classified as having the milder oedematous form of pancreatitis and 8 patients were assessed as having the severe necrotising form of the disease. No immunoreactive $D_4K$ peptide was found in samples from any of the patients with the oedematous disease, despite substantial elevation of the serum amylase in all of them, whereas $D_4K$ was identified in samples of serum and/or urine from 6 of the 8 judged to have severe pancreatitis.(Table 2)

TABLE 2

| Initial | Age | Aetiology | | | Pancreatitis | | $D_4K$ | |
|---------|-----|-----|------|-------|------|--------|-------|-------|
|         |     | GS  | Etoh | Other | Mild | Severe | Urine | Serum |
| M.B. | 76 | + |   |   |   | + | + |   |
| H.B. | 50 |   |   | + | + |   |   |   |
| J.B. | 84 | + |   |   | + |   |   |   |
| D.D. | 53 | + |   |   |   | + |   | + |
| D.G. | 70 | + |   |   | + |   |   |   |
| R.G. | 66 | + |   |   |   | + |   |   |
| W.G. | 56 |   |   | + |   | + |   | + |
| A.J. | 38 |   | + |   | + |   |   |   |
| D.K. | 84 |   |   | + | + |   |   |   |
| J.K. | 33 |   | + |   | + |   |   |   |
| P.L. | 67 |   |   | + |   | + |   |   |
| G.M. | 41 |   |   | + | + |   |   |   |
| T.N. | 63 |   |   | + | + |   |   |   |
| V.N. | 30 |   | + |   | + |   |   |   |
| I.N. | 53 | + |   |   | + |   |   |   |
| T.P. | 43 |   | + |   | + |   |   |   |
| J.P. | 68 | + |   |   | + |   |   |   |
| M.R. | 36 |   | + |   | + |   |   |   |
| A.R. | 81 | + |   |   | + |   |   |   |
| C.R. | 54 | + |   |   |   | + | + | + |
| O.S. | 48 |   |   | + |   | + |   | + |
| J.S. | 45 |   |   | + |   | + |   | + |
| TAP Assay of random samples collected at St. George's January 1985-March 1987. Stored at -20°C | | | | | | | | |

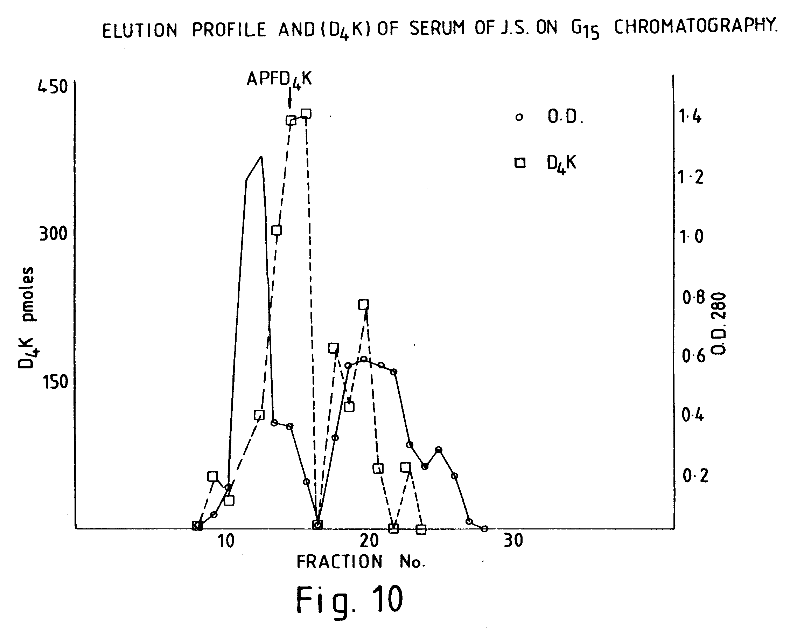

Chromatography of samples of JS sera on Sepharose G15® showed that the immunoreactivity comigrated with standard APFD$_4$K (Figure 10). In addition, serum was obtained from one patient (PD) who presented with abdominal pain and an exacerbation of chronic pancreatitis for which he had previously had a 75% distal pancreatic resection. D$_4$K peptide was detected in this sample at the level of $10^{-8}$M.

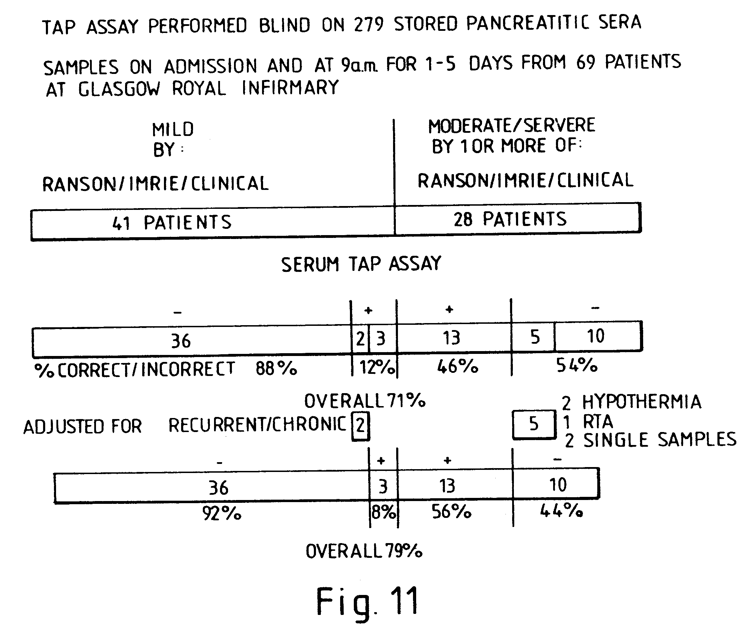

In a second clinical study assay for D$_4$K peptides was carried out on sera accumulated during routine daily sampling from 69 patients admitted to the Glasgow Royal Infirmary, Scotland, and other hospitals in the Glasgow area (Figure 11). Sera were stored frozen at -20°C and had to be thawed, aliquoted, and refrozen prior to rethawing and assay. A total of 279 stored sera were available representing serum samples taken from the 69 patients for up to 5 days, but in a few cases only a single sample was available. Assay for D$_4$K peptides was performed blind without knowledge of the severity or clinical course of the pancreatitis. 36 of 39 patients (92%) assessed as having oedematous pancreatitis were D$_4$K negative and 13 of 23 more severe cases (56%) were D$_4$K positive. The overall accuracy of the D$_4$K assay performed retrospectively on routine frozen-thawed-frozen-thawed serum-only samples in this series was 79%. Apparent false positives (3) probably reflect the known inadequacy of the clinical assessment method. Apparent false negatives (10) are likely to reflect this, together with errors introduced by the dissociation of routine once a day serum sampling from changing clinical status, and the absence of the additional information contributed by a urinary assay.

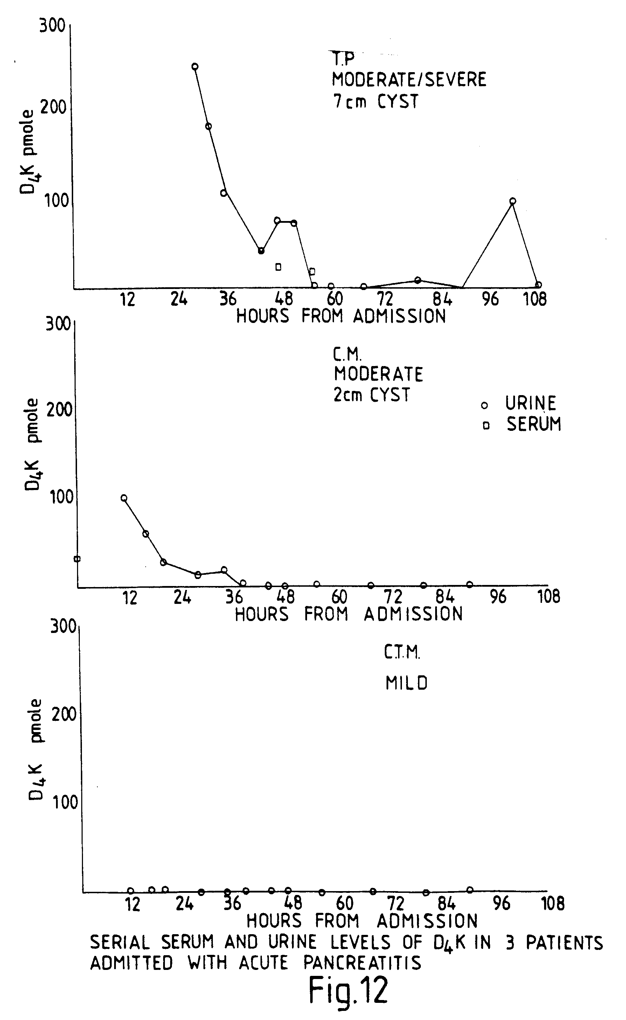

3 patients admitted to St. George's Hospital, London, SW17, England, with acute pancreatitis and hyperamylasaemia were studied using the D$_4$K assay as intended, to monitor the severity and progress of the disease. To do this, simultaneous blood and (where available) urine samples were taken immediately on admission and at 4 to 6 hourly intervals for the first 48 hours and thereafter 8 hourly. Samples were stored frozen at -20°C prior to D$_4$K assay. One patient TP (Figure 12) with moderate/severe pancreatitis but who later developed a pseudocyst, had an initial urinary D$_4$K level of 250 pmol ml$^{-1}$ 24 hours after admission, but no D$_4$K detectable in serum at this time. This reflects the look-back capability of the urine assay and the prolonged urinary excretion of D$_4$K peptide after intravenous administration identified experimentally in dogs. On the fourth day of hospitalisation, D$_4$K peptides appeared transiently in serum and later rose in urine. These changes were matched by a distinct clinical exacerbation supporting the role of the D$_4$K and other PAP assays as a precise disease-course monitor.

In a second patient CM in this series with moderate pancreatitis but with respiratory insufficiency, serum $D_4K$ was 38 pmol $ml^{-1}$ and urinary $D_4K$ 100pmol $ml^{-1}$ on admission. Both fell to zero within a few hours and thereafter remained negative, but a 2 cm pseudocyst and small lesser sac fluid collection was subsequently identified. This further supports the ability of $D_4K$ assay to predict even small areas of pancreatic necrosis. The third patient in this group, CTM, admitted with abdominal pain and a serum amylase > 7000 units, remained serum and urine $D_4K$ negative over 5 days despite his initial signs(Figure 12). He had gall stones with oedematous pancreatitis which subsequently resolved over 5 days. This again supports the role of a negative $D_4K$ assay as an accurate severity indicator in the acute disease. These results also suggest that the significant number of false negative $D_4K$ assays in the Glasgow serum-only study would not have occurred with frequent blood and urine sampling and the assay applied clinically as intended.

Additional serum samples from two patients with chronic recurrent pancreatitis in exacerbation showed $D_4K$ peptides and serum amylase levels within normal limits; this would support the diagnostic capability of the $D_4K$ assay in the chronic disease. Taken together the clinical and experimental findings with the $D_4K$ assay provide strong support for the proposed role of the invention in the diagnosis, severity prediction, and disease-course-monitoring of pancreatitis. The findings also support the principle embodied in the invention for the specific assay of body fluids for free PAP molecules using C-terminally directed antibody in pancreatic diseases.

## OTHER PAP ASSAYS OF THE INVENTION

Activation peptides of other pancreatic zymogens which are suitable for specific assay using C-terminally directed antibodies for the free species, in accordance with the present invention, include those of prophospholipase $A_2$, procolipase, proelastase 1 and proelastase 2, prekallekreins and of procarboxypeptidases A & B. Examples of these are peptides containing the carboxy terminal sequence Asp-Ser-Gly-Ile-Ser-Pro-Arg of prophospholipase $A_2$[41], peptides containing carboxy terminal Ala-Pro-Gly-Pro-Arg of procolipase [42], and peptides containing carboxy terminal Gly-Asp-Pro-Thr-Tyr-Pro-Pro-Tyr-Val-Thr-Arg of proelastase 2 [43] or peptides representing the sequence of the activation peptides of proelastase 1 or prekallekreins. Portions of these sequences representing proteolytic degradation oligopeptides of 5 or more residues such as Pro-Pro-Tyr-Val-Thr-Arg from proelastase 2 may also be used. The much larger activation peptides of procarboxypeptidases A & B may also contain oligopeptide sequences suitable as targets. The use of C-terminally directed antibody will ensure that zymogen forms normally present in human plasma and urine [57] will not be recognised in the assay as in the segregation of $D_4K$ containing free peptides from trypsinogens, already given as a detailed example.

Of additional interest and importance is the activation peptide of prophospholipase $A_2$ (PLAP). Prophospholipases $A_2$ are abundant in many cell types including macrophages [58] pulmonary alveolar macrophages [59], aggregated platelets, and polymorphnuclear leucocytes [61] as well as being generally present in lysosomes. Active phospholipase $A_2$ is released from these in several inflammatory conditions [62] including endotoxin shock [63], respiratory distress syndrome, acute abdominal conditions and in response to mycobacteria [61], peptides [60] and $Ca^{2+}$ [39]. If the prophospholipase $A_2$ from these sources is the same gene product as pancreatic prophospholipase $A_2$ and has an activation peptide of the same sequence Asp-Ser-Gly-Ile-Ser-Pro-Arg, PLAP assay as defined in this invention, will also be a useful test applicable to the recognition and severity prediction of these disorders. If the non pancreatic pro-phospholipase $A_2$ activation peptide has a sequence different from pancreatic prophospholipase $A_2$, then the PLAP assay will be pancreatic disease specific.

### Synthesis of prophospholipase $A_2$ activation peptide (PLAP) and procolipase activation peptide (CLAP)

Further examples of the invention involve the synthesis by solid-phase methods, of peptides relevant to the development of immunoassays C-terminally specific for PLAP and CLAP. These peptides are Asp-Ser-Gly-Ile-Ser-Pro-Arg, Cys-Tyr-Asp-Ser-Gly-Ile-Ser-Pro-Arg, Ala-Pro-Gly-Pro-Arg and Cys-Tyr-Ala-Pro-Gly-Pro-Arg. Syntheses were performed on p-alkoxybenzyl-alcohol-derivatised polystyrene (0.67 m equivs/g from Bachem Feinchemikalien AG, Switzerland) using temporary base-labile Fmoc/ $\alpha$-amino group protection and the general methods described by Cliffe et al [50] with the following modifications. The C-terminal residue was coupled as its protected derivative Fmoc (Mtr)Arg by a mixed anhydride method. Two equivalents of protected Arg were incubated with resin having one equivalent of alkoxybenzyl group, together with 2 equivalents of 2, 6-dichlorobenzoyl chloride and 3 of redistilled pyridine in dimethylformamide for 16h. All subsequent Fmoc amino acids were coupled in a 2-fold excess as 1-hyrdroxybenzotriazole esters. The following derivatives were employed: Fmoc-Pro, Fmoc-(OtBu)Ser, Fmoc-Ile, Fmoc-Gly, Fmoc-(OtBu)Asp, Fmoc-(OtBu)Tyr and Fmoc-(SAcM)Cys. At the end of the synthesis, the N-terminal Fmoc group was cleaved by treatment with 20% (v/v) piperidine for 10 min and the peptide cleaved and partly deprotected by treatment with 50% (v/v) trifluoroacetic acid, 5% thioanisole, 45% (v/v) dichloromethane. This was evaporated, washed four times with ether and lyophilised. Portions (250mg) of those peptides containing protected (SAcM) cysteinyl groups were extracted into minimal volumes of water and adjusted to pH4 with $NH_4OH$. Then mercuric acetate (200mg) was added and the pH readjusted to 4. After 1hr, the peptide was diluted to 50ml and $H_2S$ passed through the solution for 15 min, followed by $N_2$ for 10min. The black precipitate (HgS) was separated by centrifugation and the peptide recovered by lyophilisation.

Peptides were purified by gel chromatography on Sephadex G-15® in O.1M acetic acid and characterised by reverse phase HPLC and amino acid analysis. On Brownlee C-8 Aquapore RP-300® in 0.05% trifluoroacetic acid and acetonitrile from 5% to 25% (v/v) over 22min at 1.0ml/min peptides gave single peaks at 9.14 min for CYAPGPR, 5.49 min for APGPR. On μBondapak $C_{18}$® in 0.05% trifluoroacetic acid running and acetonitrile gradient 5% to 50% over 30 mins 1.5ml/min CYDSGISPR eluted at 11.7 min and DSGISPR at 7.8 min. Amino acid analysis confirmed the correct composition of these peptides.

Peptides with Cys-Tyr amino terminal extensions are used for haptenisation or the attachment of revealing agent or solid phase, and native peptides for use on solid phase or as competing ligands as described in the $D_4K$ example of the invention. Specific C-terminal antibodies either polyclonal or monoclonal are purified by affinity chromatography on the corresponding immobilised peptide and used in the performance of solid or solution phase assays as detailed in the $D_4K$ example. Peptides with revealing agents or immobilised on solid phase as well as specific antipeptide antibodies with or without revealing agent, and free or attached to solid phase, and the use of such reagents in the performance of PAP assays in the diagnosis and severity monitoring of disease form further aspects of the invention.

BIBLIOGRAPHY

1.  Corfield A.P. et al (1985). Gut 26:724-729.
2.  Hermon-Taylor J and Heywood G.C. (1985). Scand.J.Gastroenterol.20(Suppl.117), 39-46.
3.  Salt W.B. and Schenker S. (1976). Medicine 55:269-289.
4.  Temler R.S. and Felber J-P. (1976). Biochim. Biophys. Acta. 445:720-728.
5.  Elias E. et al (1977). Lancet ii:66-68.
6.  Borgstrom A. and Ohlsson K. (1978). Hoppe-Seyler's Z. Physiol. Chem. 359:677-681.
7.  Adrian T.E. et al (1979). Clin Chim Acta 97:205-212.
8.  Lake-Bakaar G. et al (1979). Lancet ii:878-880.
9.  Crossley J.R. et al (1979). Lancet i:472-474.
10.  Brodrick J.W. et al (1979). Am. J. Physiol. 237:E474-E480.
11.  Geokas M.C. et al (1979). Am. J. Physiol. 236:E77-E83.
12.  Koop H. et al (1980). Digestion 20:151-156.
13.  Duffy M.J. et al (1980). Clin. Chim. Acta. 103:233-235.
14.  Malvano R. et al (1980). Scand. J. Gastroenterol. 15(suppl. 62):3-62.
15.  Geokas M.C. et al (1981). Am. J. Pathol. 105:31-39.
16.  Farini R. et al (1981). Gastroenterology 81:242-246.
17.  Masoero G. et al (1982). Dig. Dis. Sci. 27:1089-1094.
18.  Steinberg W.M. and Anderson K.K. (1984). Dig. Dis. Sci. 29:988-993.
19.  Umeki S. et al (1985). J. Lab. Clin. Med. 106:578-582.
20.  Wellborn J.C. et al (1983). Am. J. Surg. 146:834-837.
21.  Geokas M.C. et al (1979). J.Biol. Chem.254:2775-2781.
22.  Eskola J.U. et al (1983). Clin. Chem. 29:1777-1780.
23.  Nevalainen T.J. et al (1985). Clin. Chem. 31:1116-1120.
24.  Delk A.S. et al (1985). Clin.Chem. 31:1294-1300.
25.  Goldberg J.M. (1976). Clin. Chem. 22:638-642.
26.  Mayer A.D. et al (1985). Br. J. Surg. 72:436-437.
27.  Kitahara T. et al (1980). Clin. Chim. Acta. 103:135-143.
28.  Otsuki M. et al (1984). Clin. Chim. Acta. 142:231-240.
29.  O'Connor C.M. et al (1981). Clin. Chim. Acta. 114:29-35.
30.  Hermon-Taylor J. et al (1981). Clin. Chim. Acta. 109:203-209.
31.  Massey T.H. (1985). Clin. Chem. 31:70-75.

32. Pace B.W. et al (1985). Am. J. Gastroenterol. 80:898-901.
33. Mifflin T.E. et al (1985). Clin. Chem. 31:1283-1288.
34. Moller-Petersen J. et al (1986). Clin. Chim. Acta. 157:151-166.
35. DiMagno E.P. (1983). Alabama J. Med. Sci. 20:404-410.
36. Spechler S.J. et al (1983). Dig. Dis. Sci. 28:865-869.
37. Eckfeldt J.H. et al (1985). Arch. Pathol. Lab. Med. 109:316-319.
38. Puolakkainen P.et al (1987). Gut. 28:764-771.
39. Traynor J.R. et al (1981). Biochim. Biophys.Acta. 665:571-577.
40. Vadas P. (1984). J. Lab.Clin.Med. 104:873-881.
41. Grataroli R. et al (1982). Eur. J.Biochem. 122:111-117.
42. Sternby B. et al (1984). Biochim. Biophys. Acta. 786:109-112.
43. Largman C. et al (1980). Biochim. Biophys. Acta. 623:208-212.
44. Maroux S. et al (1971). J. Biol. Chem. 246:5031-5039.
45. Broderick J.W. et al (1978). J. Biol. Chem. 253:2737-2742.
46. Stanley C.J. et al (1985). J. Immunol. Methods 83:89-95.
47. Carpino L.A. and Han G.Y. (1972). J. Org. Chem. 37:3404-3409.
48. Wang S. (1973). J. Amer. Chem. Soc. 95:1328-1333.
49. Meienhofer J. et al (1979). Int. J. Pep. Prot. Res. 13:35-42.
50. Cliffe S.G.R. et al (1985). Int. J. Pep. Prot. Res.25:663-672.
51. Bassiri R.M. and Utiger R.D. (1972). Endocrinology 90:722-727.
52. Green N. et al (1982). Cell 28:477-484.
53. Hoffman W. et al EMBO. J. (1983). 2:711-714.
54. Cawthorne S.J. et al (1984). Dig. Dis. Sci. 29:945.
55. Shorrock K. et al (1986). Gut 27:A1260.
56. Ranson J.H.C. et al (1974). S.G.O. 139:69-81.
57. Sternby B. et al (1984). Biochim. Biophys. Acta. 789:164-69.
58. Vadas P. and Hay J.B. (1980). Life Sci. 26:1721-29.
59. Lanni C. et al (1981). Biochim. Biophys. Act. 658:54-63.
60. Lanni C. et al (1983). Am. J. Pathol. 113:90-94.
61. Franson R.C. et al (1973). J. Cell. Biol. 56:621-627.
62. Franson R.C. et al (1978). J. Lipid. Res. 19:18-23.
63. Vadas P. (1984). J. Lab.Clin.Med. 104:873-881.

## Claims

**Claims for the following Contracting States : AT, BE, CH, DE, FR, GB, IT, LI, LU, NL, SE**

1. A method for diagnosing pancreatic disease in a patient which comprises assaying a sample of the patient's body fluid for the presence or absence of peptides which are the activation peptides of pancreatic zymogens specifically cleaved by proteolysis during activation (PAP).

2. A method according to claim 1 wherein the peptide assayed for is a trypsinogen activation peptide (TAP) comprising the amino acid sequence $D_4 K$ having the lysine (K) as the carboxy terminus.

3. A method according to claim 1 wherein the peptide assayed for is human pancreatic prophospholipase A2 activation peptide (PLAP) comprising the amino acid sequence

D S G I S P R

having the arginine (R) as the carboxy terminus

or procolipase activation peptide (CLAP) comprising the amino acid sequence

A P G P R

having the arginine (R) as the carboxy terminus

or proelastase 2 activation peptide (PEAP) comprising the amino acid sequence

G D P T Y P P Y V T R

having the arginine (R) as the carboxy terminus or the degradation product of proelastase 2 activation peptide (PEAP) comprising the amino acid sequence

P P Y V T R

having the arginine (R) as the carboxy terminus.

EP 0 258 995 B1

**4.** A method according to any one of the preceding claims wherein the body fluid is blood, ascites or urine.

**5.** A method according to any one of the preceding claims wherein the body fluid is brought into contact with a C-terminally directed antibody having specificity for PAP.

**6.** A method according to claim 5 wherein the antibody has specificity for TAP, PLAP, CLAP or PEAP.

**7.** A method according to claim 5 or 6 involving the formation of a conjugate between the PAP and the antibody, said conjugate carrying a revealing label and being formed either in the solid phase or in the liquid phase of a solid/liquid phase reaction mixture, separating the solid phase from the liquid phase and determining the presence of or amount of the revealing label in either the solid phase or the liquid phase as a measure of the presence of or amount of, respectively, PAP in the sample.

**8.** A method according to claim 7 carried out as an ELISA or using biotin labelled antibody.

**9.** A method according to claim 7 carried out as an ELISA or using biotin labelled PAP.

**10.** A process according to any one of the preceding claims wherein the sample is brought into contact with a solid phase comprising an inert solid support to which is bound
    either (1) PAP having bound to it an antibody to PAP, the antibody carrying a revealing label,
    or (2) antibody to PAP having bound to it PAP, the PAP carrying a revealing label.

**11.** A method according to any one of the preceding claims wherein samples of body fluid are removed from the patient on at least two separate occasions spaced apart from one another by at least 2 hours and each sample is assayed for the concentration of PAP as a means for monitoring the severity progress of pancreatitis.

**12.** The pentapeptide $D_4 K$ having a revealing label directly attached to one of its constituent amino acids.

**13.** The peptide
    D S G I S P R
or
    A P G P R
or
    G D P T Y P P Y V T R
or
    P P Y V T R
having a revealing label directly attached to one of its constituent amino acids.

**14.** A polypeptide including the peptide of claim 12 or 13 conjugated with another polypeptide.

**15.** The peptide
    $D_4 K$
or
    D S G I S P R
or
    A P G P R
or
    G D P T Y P P Y V T R
or
    P P Y V T R
coupled at its amino terminus to a protein carrier.

**16.** A C-terminally directed antibody having specificity for PAP.

**17.** An antibody according to claim 16 having specificity for TAP, PLAP, CLAP or PEAP.

15

**18.** A monoclonal antibody according to any one of claims 16 or 17.

**19.** An antibody according to claim 16 or 17 isolated from the serum of an animal immunised with an immunogen including a PAP amino acid sequence.

**20.** An antibody according to any one of claims 16 to 19 having a revealing label.

**21.** A peptide or polypeptide according to any one of claims 12 to 15 immobilised on an inert solid support, the revealing label being optionally absent.

**22.** An antibody according to any one of claims 10 to 20 immobilised on an inert solid support.

**23.** A solid component for a diagnostic test kit comprising a peptide or polypeptide according to claim 21 to which peptide or polypeptide an antibody according to claim 20 is bound.

**24.** A solid component for a diagnostic test kit comprising an antibody according to claim 22 to which a peptide or polypeptide according to any one of claims 12 to 15 is bound.

**25.** A diagnostic test kit comprising, as one component, a labelled peptide or polypeptide according to any one of claims 12 to 14, 21 or 24 or labelled antibody according to any one of claims 20, 22 or 23.

**Claims for the following Contracting States : ES, GR**

**1.** A method for diagnosing pancreatic disease in a patient which comprises assaying a sample of the patient's body fluid for the presence or absence of peptides which are the activation peptides of pancreatic zymogens specifically cleaved by proteolysis during activation (PAP).

**2.** A method according to claim 1 wherein the peptide assayed for is a trypsinogen activation peptide (TAP) comprising the amino acid sequence $D_4 K$ having the lysine (K) as the carboxy terminus.

**3.** A method according to claim 1 wherein the peptide assayed for is human pancreatic prophospholipase A2 activation peptide (PLAP) comprising the amino acid sequence
    D S G I S P R
having the arginine (R) as the carboxy terminus
    or procolipase activation peptide (CLAP) comprising the amino acid sequence
    A P G P R
    having the arginine (R) as the carboxy terminus
    or proelastase 2 activation peptide (PEAP) comprising the amino acid sequence
    G D P T Y P P Y V T R
having the arginine (R) as the carboxy terminus or the degradation product of proelastase 2 activation peptide (PEAP) comprising the amino acid sequence
    P P Y V T R
having the arginine (R) as the carboxy terminus.

**4.** A method according to any one of the preceding claims wherein the body fluid is blood, ascites or urine.

**5.** A method according to any one of the preceding claims wherein the body fluid is brought into contact with a C-terminally directed antibody having specificity for PAP.

**6.** A method according to claim 5 wherein the antibody has specificity for TAP, PLAP, CLAP or PEAP.

**7.** A method according to claim 5 or 6 involving the formation of a conjugate between the PAP and the antibody, said conjugate carrying a revealing label and being formed either in the solid phase or in the liquid phase of a solid/liquid phase reaction mixture, separating the solid phase from the liquid phase and determining the presence of or amount of the revealing label in either the solid phase or the liquid phase as a measure of the presence of or amount of, respectively, PAP in the sample.

16

EP 0 258 995 B1

8. A method according to claim 7 carried out as an ELISA or using biotin labelled antibody.

9. A method according to claim 7 carried out as an ELISA or using biotin labelled PAP.

10. A process according to any one of the preceding claims wherein the sample is brought into contact with a solid phase comprising an inert solid support to which is bound
either (1) PAP having bound to it an antibody to PAP, the antibody carrying a revealing label,
or (2) antibody to PAP having bound to it PAP, the PAP carrying a revealing label.

11. A method according to any one of the preceding claims wherein samples of body fluid are removed from the patient on at lease two separate occasions spaced apart from one another by at least 2 hours and each sample is assayed for the concentration of PAP as a means for monitoring the severity progress of pancreatitis.

12. A method for making a labelled peptide for use in the detection of pancreatic zymogens which comprises attaching a revealing label directly to one of the constituent amino acids of the peptide:
$DK_4$,
or
D S G I S P R,
or
A P G P R,
or
G D P T Y P P Y V T R,
or
P P Y V T R.

13. A method of making a polypeptide for use in the detection of pancreatic zymogens which comprises conjugating a polypeptide including the peptide of claim 12 with another polypeptide.

14. A method according to claim 12 or 13 which further comprises immobilizing the peptide on an inert solid support, the revealing label being optionally absent.

15. A method of making a labelled antibody for use in the detection pancreatic zymogens which comprises conjugating a C-terminally directed antibody having specificity for PAP with a revealing label.

16. A method according to claim 15 wherein the antibody has specificity for TAP, PLAP, CLAP OR PEAP.

17. A method according to claim 15 or 16 which further comprises immobilizing the antibody on an inert solid support.

**Patentansprüche**
**Patentansprüche für folgende Vertragsstaaten : AT, BE, CH, DE, FR, GB, IT, LI, LU, NL, SE**

1. Verfahren zur Diagnose einer pankreatischen Erkrankung bei einem Patienten, welches das Analysieren einer Probe von Körperflüssigkeit des Patienten auf die Gegenwart oder Abwesenheit von Peptiden umfaßt, welche die Aktivierungs-Peptide pankreatischer Zymogene sind, die spezifisch durch Proteolyse während der Aktivierung (PAP) aufgespalten werden.

2. Verfahren nach Anspruch 1, worin das Zielpeptid der Analyse ein Trypsinogen-Aktivierungspeptid (TAP) ist, umfassend die Aminosäuresequenz $D_4 K$, wobei das Lysin (K) der Carboxy-Terminus ist.

3. Verfahren nach Anspruch 1, worin das Zielpeptid der Analyse menschliches pankreatisches Prophospholipase A2-Aktivierungspeptid (PLAP) ist, umfassend die Aminosäuresequenz
D S G I S P R,
wobei das Arginin (R) der Carboxy-Terminus ist,
oder Procolipase-Aktivierungspeptid (CLAP) ist,
umfassend die Aminosäuresequenz
A P G P R,

17

wobei das Arginin (R) der Carboxy-Terminus ist,
   oder Proelastase 2-Aktivierungspeptid (PEAP) ist,
umfassend die Aminosäuresequenz
   G D P T Y P P Y V T R,
wobei das Arginin (R) der Carboxy-Terminus ist,
   oder das Abbauprodukt des Proelastase 2-Aktivierungspeptid (PEAP) ist,
umfassend die Aminosäuresequenz
   P P Y V T R,
wobei das Arginin (R) der Carboxy-Terminus ist.

4. Verfahren nach einem der vorangegangenen Ansprüche, worin die Kõrperflüssigkeit Blut, Aszites oder Urin ist.

5. Verfahren nach einem der vorangegangenen Ansprüche, worin die Kõrperflüssigkeit in Berührung mit einem C-terminai gerichteten Antikõrper gebracht wird, der für PAP spezifisch ist.

6. Verfahren nach Anspruch 5, worin der Antikõrper spezifisch für TAP, PLAP, CLAP oder PEAP ist.

7. Verfahren nach Anspruch 5 oder 6, umfassend das Bilden eines Konjugats zwischen dem PAP und dem Antikõrper, wobei das Konjugat eine Erkennungsmarkierung trägt und entweder in der Festphase oder in der Flüssigphase einer Fest/Flüssigphasen-Reaktionsmischung gebildet wird, das Trennen der Festphase von der Flüssigphase und das Bestimmen der Gegenwart von oder der Menge an Erkennungsmarkierung entweder in der Festphase oder in der Flüssigphase als jeweils einem Maß für die Gegenwart von oder der Menge an PAP in der Probe.

8. Verfahren nach Anspruch 7, das als ein ELISA oder unter Verwendung von mit Biotin markiertem Antikõrper durchgeführt wird.

9. Verfahren nach Anspruch 7, das als ein ELISA oder unter Verwendung von mit Biotin markiertem PAP durchgeführt wird.

10. Verfahren nach einem der vorangegangenen Ansprüche, worin die Probe mit einer Festphase in Berührung gebracht wird, umfassend ein inertes Trägermaterial, an welches gebunden ist:
   entweder (1) PAP, an das ein Antikõrper zu PAP gebunden ist, wobei der Antikõrper eine Erkennungsmarkierung trägt,
   oder (2) Antikõrper zu PAP, an den PAP gebunden ist, wobei das PAP eine Erkennungsmarkierung trägt.

11. Verfahren nach einem der vorangegangenen Ansprüche, worin dem Patienten Proben von Kõrperflüssigkeit bei mindestens zwei verschiedenen Gelegenheiten, die zeitlich mindestens 2 Stunden auseinanderliegen, entnommen wird und jede Probe auf die Konzentration des PAP analysiert wird als einer Maßnahme zur Überwachung des Fortschreitens des Schweregrades der Pankreatitis.

12. Pentapeptid $D_4K$, bei dem eine Erkennungsmarkierung direkt auf einem seiner Aminosäure-Bestandteile angebracht ist.

13. Peptid
   D S G I S P R
oder
   A P G P R
oder
   G D P T Y P P Y V T R
oder
   P P Y V T R,
bei dem eine Erkennungsmarkierung direkt auf einem seiner Aminosäure-Bestandteile angebracht ist.

14. Polypeptid, einschließlich des Peptids nach Anspruch 12 oder 13, das mit einem anderen Polypeptid konjugiert ist.

EP 0 258 995 B1

**15.** Peptid

    $D_4$ K

oder

    D S G I S P R

oder

    A P G P R

oder

    G D P T Y P P Y V T R

oder

    P P Y V T R,

das an seinem Amino-Terminus an einen Proteinträger gekoppelt ist.

**16.** C-terminal gerichteter Antikõrper, der für PAP spezifisch ist.

**17.** Antikõrper nach Anspruch 16, der für TAP, PLAP, CLAP oder PEAP spezifisch ist.

**18.** Monoklonaler Antikõrper nach einem der Ansprüche 16 oder 17.

**19.** Antikõrper nach Anspruch 16 oder 17, welcher aus dem Serum eines Tieres isoliert ist, das mit einem Immunogen immunisiert ist, und eine PAP-Aminosãuresequenz enthãlt.

**20.** Antikõrper nach einem der Ansprüche 16 bis 19, der eine Erkennungsmarkierung trãgt.

**21.** Peptid oder Polypeptid nach einem der Ansprüche 12 bis 15, das auf einem inerten Trãgermaterial immobilisiert ist, wobei die Erkennungsmarkierung wahlweise abwesend ist.

**22.** Antikõrper nach einem der Ansprüche 16 bis 20, der auf einem inerten Trãgermaterial immobilisiert ist.

**23.** Festbestandteil für eine diagnostische Testpackung, umfassend ein Peptid oder Polypeptid nach Anspruch 21, an welches Peptid oder Polypeptid ein Antikõrper nach Anspruch 20 gebunden ist.

**24.** Festbestandteil für eine diagnostische Testpackung, umfassend einen Antikõrper nach Anspruch 22, an welchen ein Peptid oder Polypeptid nach einem der Ansprüche 12 bis 15 gebunden ist.

**25.** Diagnostische Testpackung, welche als einen der Bestandteile ein markiertes Peptid oder Polypeptid nach einem der Ansprüche 12 bis 14, 21 oder 24, oder einen markierten Antikõrper nach einem der Ansprüche 20, 22 oder 23 umfaßt.

**Patentansprüche für folgende Vertragsstaaten : ES, GR**

**1.** Verfahren zur Diagnose einer pankreatischen Erkrankung bei einem Patienten, welches das Analysieren einer Probe von Kõrperflüssigkeit des Patienten auf die Gegenwart oder Abwesenheit von Peptiden umfaßt, welche die Aktivierungs-Peptide pankreatischer Zymogene sind, die spezifisch durch Proteolyse wãhrend der Aktivierung (PAP) aufgespalten werden.

**2.** Verfahren nach Anspruch 1, worin das Zielpeptid der Analyse ein Trypsinogen-Aktivierungspeptid (TAP) ist, umfassend die Aminosãuresequenz $D_4$ K, wobei das Lysin (K) der Carboxy-Terminus ist.

**3.** Verfahren nach Anspruch 1, worin das Zielpeptid der Analyse menschliches pankreatisches Prophospholipase A2-Aktivierungspeptid (PLAP) ist, umfassend die Aminosãuresequenz

    D S G I S P R,

wobei das Arginin (R) der Carboxy-Terminus ist,

    oder Procolipase-Aktivierungspeptid (CLAP) ist,

umfassend die Aminosãuresequenz

    A P G P R,

wobei das Arginin (R) der Carboxy-Terminus ist,

    oder Proelastase 2-Aktivierungspeptid (PEAP) ist,

umfassend die Aminosãuresequenz

19

G D P T Y P P Y V T R,

wobei das Arginin (R) der Carboxy-Terminus ist,

oder das Abbauprodukt des Proelastase 2-Aktivierungspeptid (PEAP) ist,

umfassend die Aminosäuresequenz

P P Y V T R,

wobei das Arginin (R) der Carboxy-Terminus ist.

4. Verfahren nach einem der vorangegangenen Ansprüche, worin die Kõrperflüssigkeit Blut, Aszites oder Urin ist.

5. Verfahren nach einem der vorangegangenen Ansprüche, worin die Kõrperflüssigkeit in Berührung mit einem C-terminal gerichteten Antikõrper gebracht wird, der für PAP spezifisch ist.

6. Verfahren nach Anspruch 5, worin der Antikõrper spezifisch für TAP, PLAP, CLAP oder PEAP ist.

7. Verfahren nach Anspruch 5 oder 6, umfassend das Bilden eines Konjugats zwischen dem PAP und dem Antikõrper, wobei das Konjugat eine Erkennungsmarkierung trägt und entweder in der Festphase oder in der Flüssigphase einer Fest/Flüssigphasen-Reaktionsmischung gebildet wird, das Trennen der Festphase von der Flüssigphase und das Bestimmen der Gegenwart von oder der Menge an Erkennungsmarkierung entweder in der Festphase oder in der Flüssigphase als jeweils einem Maß für die Gegenwart von oder der Menge an PAP in der Probe.

8. Verfahren nach Anspruch 7, das als ein ELISA oder unter Verwendung von mit Biotin markiertem Antikõrper durchgeführt wird.

9. Verfahren nach Anspruch 7, das als ein ELISA oder unter Verwendung von mit Biotin markiertem PAP durchgeführt wird.

10. Verfahren nach einem der vorangegangenen Ansprüche, worin die Probe mit einer Festphase in Berührung gebracht wird, umfassend ein inertes Trägermaterial, an welches gebunden ist:

entweder (1) PAP, an das ein Antikõrper zu PAP gebunden ist, wobei der Antikõrper eine Erkennungsmarkierung trägt,

oder (2) Antikõrper zu PAP, an den PAP gebunden ist, wobei das PAP eine Erkennungsmarkierung trägt.

11. Verfahren nach einem der vorangegangenen Ansprüche, worin dem Patienten Proben von Kõrperflüssigkeit bei mindestens zwei verschiedenen Gelegenheiten, die zeitlich mindestens 2 Stunden auseinanderliegen, entnommen wird und jede Probe auf die Konzentration des PAP analysiert wird als einer Maßnahme zur Überwachung des Fortschreitens des Schweregrades der Pankreatitis.

12. Verfahren zur Herstellung eines markierten Peptids zur Verwendung beim Nachweisen pankreatischer Zymogene, umfassend das Anbringen der Erkennungsmarkierung direkt auf einem der Aminosäure-Bestandteile des Peptids

D K

oder

D S G I S P R

oder

A P G P R

oder

G D P T Y P P Y V T R

oder

P P Y V T R.

13. Verfahren zur Herstellung eines Polypeptids zur Verwendung beim Nachweisen pankreatischer Zymogene, welches das Konjugieren eines Polypeptids, einschließlich des Peptids nach Anspruch 12, mit einem anderen Polypeptid umfaßt.

**EP 0 258 995 B1**

14. Verfahren nach Anspruch 12 oder 13, welches weiterhin das Immobilisieren des Peptids auf einem inerten Trägermaterial umfaßt, wobei die Erkennungsmarkierung wahlweise abwesend ist.

15. Verfahren zur Herstellung eines markierten Antikõrpers zur Verwendung beim Nachweisen pankreatischer Zymogene, welches das Konjugieren eines C-terminal gerichteten Antikõrpers, der für PAP spezifisch ist, mit einer Erkennungsmarkierung umfaßt.

16. Verfahren nach Anspruch 15, worin der Antikörper für TAP, PLAP, CLAP oder PEAP spezifisch ist.

17. Verfahren nach Anspruch 15 oder 16, das weiterhin das Immobilisieren des Antikõrpers auf einem inerten Trägermaterial umfaßt.

**Revendications**
**Revendications pour les Etats contractants suivants : AT, BE, CH, DE, FR, GB, IT, LI, LU, NL, SE**

1. Méthode permettant de diagnostiquer une maladie du pancréas chez un patient, comprenant la recherche, dans un échantillon de fluide corporel du patient, de la présence ou de l'absence de peptides qui sont les peptides résultant de l'activation de zymogènes pancréatiques clivés spécifiquement par protéolyse lors de l'activation (PAP).

2. Méthode selon la revendication 1, dans laquelle le peptide recherché est un peptide résultant de l'activation du trypsinogène (TAP) comprenant la séquence d'acides aminés $D_4K$, la lysine (K) constituant l'extrémité carboxyterminale.

3. Méthode selon la revendication 1 dans laquelle le peptide recherché est le peptide résultant de l'activation de la prophospholipase pancréatique humaine A2 (PLAP) comportant la séquence d'acides aminés
    DSGISPR,
l'arginine (R) constituant l'extrémité carboxyterminale
    ou le peptide résultant de l'activation de la procolipase (CLAP) comportant la séquence d'acides aminés
    APGPR,
l'arginine (R) constituant l'extrémité carboxyterminale
    ou le peptide résultant de l'activation de la proélastase 2 (PEAP) comportant la séquence d'acides aminés
    GDPTYPPYVTR,
l'arginine (R) constituant l'extrémité carboxyterminale
    ou le produit de dégradation du peptide résultant de l'activation de la proélastase 2 (PEAP) comportant la séquence d'acides aminés
    PPYVTR,
l'arginine (R) constituant l'extrémité carboxyterminale.

4. Méthode selon l'une quelconque des revendications précédentes dans laquelle le fluide corporel est du sang, des ascites ou de l'urine.

5. Méthode selon l'une quelconque des revendications précédentes dans laquelle le fluide corporel est mis en contact avec un anticorps dirigé contre l'extrémité carboxyterminale spécifique du PAP.

6. Méthode selon la revendication 5 dans laquelle l'anticorps est spécifique du TAP, PLAP, CLAP ou PEAP.

7. Méthode selon la revendication 5 ou 6 comprenant la formation d'un conjugué entre le PAP et l'anticorps, ledit conjugué portant un marqueur de détection et étant formé soit dans la phase solide soit dans la phase liquide d'un mélange réactionnel constitué d'une phase solide et d'une phase liquide, la séparation de la phase solide de la phase liquide et la détermination de la présence de ou de la quantité de marqueur de détection, soit dans la phase solide soit dans la phase liquide, en tant que mesure, respectivement, de la présence ou de la quantité de PAP dans l'échantillon.

21

EP 0 258 995 B1

**8.** Méthode selon la revendication 7 réalisée sous forme d'ELISA ou en utilisant un anticorps marqué à la biotine.

**9.** Méthode selon la revendication 7 réalisée sous forme d'ELISA ou en utilisant du PAP marqué à la biotine.

**10.** Procédé selon l'une quelconque des revendications précédentes, dans lequel l'échantillon est mis en contact avec une phase solide comprenant un support solide inerte, auquel est attaché
soit (1) du PAP auquel est lié un anticorps dirigé contre le PAP, l'anticorps portant un marqueur de détection,
ou (2) un anticorps dirigé contre le PAP auquel est lié du PAP, le PAP portant un marqueur de détection.

**11.** Méthode selon l'une quelconque des revendications précédentes dans laquelle on prélève, au moins à deux reprises, des échantillons de fluide corporel du patient, les deux prélèvements étant espacés l'un de l'autre d'au moins deux heures, et on mesure la concentration de PAP dans chaque échantillon afin de suivre l'aggravation de la pancréatite.

**12.** Pentapeptide $D_4K$ doté d'un marqueur de détection, fixé directement à l'un des acides aminés qui le constituent.

**13.** Peptide
DSGISPR
ou
APGPR
ou
GDPTYPPYVTR
ou
PPYVTR
doté d'un marqueur de détection, fixé directement à l'un des acides aminés qui le constituent.

**14.** Polypeptide comprenant le peptide de la revendication 12 ou 13 conjugué avec un autre polypeptide.

**15.** Peptide
$D_4K$
ou
DSGISPR
ou
APGPR
ou
GDPTYPPYVTR
ou
PPYVTR
couplé à son extrémité aminoterminale à un véhicule protéique.

**16.** Anticorps dirigé contre l'extrémité C-terminale spécifique du PAP.

**17.** Anticorps selon la revendication 16 spécifique du TAP, PLAP, CLAP ou PEAP.

**18.** Anticorps monoclonal selon l'une quelconque des revendications 16 ou 17.

**19.** Anticorps selon la revendication 16 ou 17 isolé à partir du sérum d'un animal immunisé avec une substance immunogène comprenant la séquence d'acides aminésde PAP.

**20.** Anticorps selon l'une quelconque des revendications 16 à 19, doté d'un marqueur de détection.

**21.** Peptide ou polypeptide selon l'une quelconque des revendications 12 à 15, immobilisé sur un support solide inerte, le marqueur de détection pouvant être omis.

22

EP 0 258 995 B1

**22.** Anticorps selon l'une quelconque des revendications 16 à 20, immobilisé sur un support solide inerte.

**23.** Composant solide destiné au kit de diagnostic comprenant un peptide ou un polypeptide selon la revendication 21, auquel est lié un anticorps selon la revendication 20.

**24.** Composant solide destiné au kit de diagnostic comprenant un anticorps selon la revendication 22, auquel est lié un peptide ou un polypeptide selon l'une quelconque des revendications 12 à 15.

**25.** Kit de diagnostic dont l'un des composants est constitué d'un peptide ou d'un polypeptide marqué selon l'une quelconque des revendications 12 à 14, 21 ou 24 ou d'un anticorps marqué selon l'une quelconque des revendications 20, 22 ou 23.

**Revendications pour les Etats contractants suivants : ES, GR**

**1.** Méthode permettant de diagnostiquer une maladie du pancréas chez un patient, comprenant la recherche, dans un échantillon de fluide corporel du patient, de la présence ou de l'absence de peptides qui sont les peptides résultant de l'activation de zymogènes pancréatiques clivés spécifiquement par protéolyse lors de l'activation (PAP).

**2.** Méthode selon la revendication 1, dans laquelle le peptide recherché est un peptide résultant de l'activation du trypsinogène (TAP) comprenant la séquence d'acides aminés $D_4K$, la lysine (K) constituant l'extrémité carboxyterminale.

**3.** Méthode selon la revendication 1 dans laquelle le peptide recherché est le peptide résultant de l'activation de la prophospholipase pancréatique humaine A2 (PLAP) comportant la séquence d'acides aminés
DSGISPR,
l'arginine (R) constituant l'extrémité carboxyterminale
ou le peptide résultant de l'activation de la procolipase (CLAP) comportant la séquence d'acides aminés
APGPR,
l'arginine (R) constituant l'extrémité carboxyterminale
ou le peptide résultant de l'activation de laproélastase 2 (PEAP) comportant la séquence d'acides aminés
GDPTYPPYVTR,
l'arginine (R) constituant l'extrémité carboxyterminale
ou le produit de dégradation du peptide résultant de l'activation de la proélastase 2 (PEAP) comportant la séquence d'acides aminés
PPYVTR,
l'arginine (R) constituant l'extrémité carboxyterminale.

**4.** Méthode selon l'une quelconque des revendications précédentes dans laquelle le fluide corporel est le sang, des ascites ou de l'urine.

**5.** Méthode selon l'une quelconque des revendications précédentes dans laquelle le fluide corporel est mis en contact avec un anticorps spécifique dirigé contre l'extrémité carboxyterminale de PAP.

**6.** Méthode selon la revendication 5 dans laquelle l'anticorps est spécifique du TAP, PLAP, CLAP ou PEAP.

**7.** Méthode selon la revendication 5 ou 6 comprenant la formation d'un conjugué entre le PAP et l'anticorps, ledit conjugué portant un marqueur de détection et étant formé soit dans la phase solide soit dans la phase liquide d'un mélange réactionnel constitué d'une phase solide et d'une phase liquide, la séparation de la phase solide de la phase liquide et la détermination de la présence de ou de la quantité de marqueur de détection, soit dans la phase solide soit dans la phase liquide, en tant que mesure, respectivement, de la présence ou de la quantité de PAP dans l'échantillon.

23

8. Méthode selon la revendication 7 réalisée sous forme d'ELISA ou en utilisant un anticorps marqué à la biotine.

9. Méthode selon la revendication 7 réalisée sous forme d'ELISA ou en utilisant du PAP marqué à la biotine.

10. Procédé selon l'une quelconque des revendications précédentes, dans lequel l'échantillon est mis en contact avec une phase solide comprenant un support solide inerte, auquel est attaché
    soit (1) du PAP auquel est lié un anticorps dirigé contre le PAP, l'anticorps portant un marqueur de détection,
    ou (2) un anticorps dirigé contre le PAP auquel est lié du PAP, le PAP portant un marqueur de détection.

11. Méthode selon l'une quelconque des revendications précédentes dans laquelle on prélève, au moins à deux reprises, des échantillons de fluide corporel du patient, les deux prélèvements étant espacés l'un de l'autre d'au moins deux heures, et on mesure la concentration de PAP dans chaque échantillon afin de suivre l'aggravation de la pancréatite.

12. Méthode de production d'un peptide marqué, pour l'utilisation dans la détection de zymogènes pancréatiques, comprenant la fixation d'un marqueur de détection, directement à l'un des acides aminés qui constituent le peptide:
    DK4,
    ou
    DSGISPR,
    ou
    APGPR,
    ou
    GDPTYPPYVTR,
    ou
    PPYVTR,

13. Méthode de production d'un polypeptide pour l'utilisation dans la détection de zymogènes pancréatiques, comprenant la conjugaison d'un polypeptide comportant le peptide de la revendication 12 avec un autre polypeptide.

14. Méthode selon la revendication 12 ou 13, comprenant, en outre, l'immobilisation du peptide sur un support solide inerte, le marqueur de détection pouvant être omis.

15. Méthode de production d'un anticorps marqué pour l'utilisation dans la détection de zymogènes pancréatiques, comprenant la conjugaison d'un anticorps dirigé contre l'extrémité C-terminale spécifique du PAP avec un marqueur de détection.

16. Méthode selon la revendication 15, dans laquelle l'anticorps est spécifique du TAP, PLAP CLAP ou PEAP.

17. Méthode selon la revendication 15 ou 16, comprenant, en outre, l'immobilisation de l'anticorps sur un support solide inerte.

Fig. 1

EFFECT OF VARIOUS CALCIUM CONCENTRATIONS ON BINDING TO $^{125}I$-LABELLED $YD_4K$ IN SERA FROM 3 RABBITS

o $R_2$
□ $R_{17}$
◇ $R_{18}$

% BOUND

LOG mM ($Ca^{2+}$)

EP 0 258 995 B1

ELUTION PROFILES OF ANTI-$D_4K$ ANTISERA FROM RABBITS 2 AND 17 AFTER AFFINITY CHROMATOGRAPHY ON IMMOBILISED $D_4K$, SHOWING THE PRESENCE OF $Ca^{2+}$ DEPENDENT AND $Ca^{2+}$ INDEPENDENT ANTIBODIES.

Fig. 2

DISPLACEMENT BY SYNTHETIC PEPTIDES OF $^{125}$I-LABELLED YD$_4$K
BOUND TO SPECIFIC ANTISERA.

Fig. 3

EP 0 258 995 B1

5 DISPLACEMENT OF BINDING BY $^{125}$I-YD$_4$K BY TRYPSINOGEN (PURIFIED AND IN PANCREATIC JUICE) WITH AND WITHOUT ENTEROKINASE ACTIVATION

■—■ TRYPSINOGEN
●—● TRYPSINOGEN + EK
○—○ PANCREATIC JUICE
▲—▲ PANCREATIC JUICE + EK

Fig. 4

EP 0 258 995 B1

EP 0 258 995 B1

Fig.5

ELUTION PROFILE OF FROG SKIN SECRETION FROM C-8 REVERSE PHASE COLUMN

IMMUNOREACTIVITY IN C-8 FRACTIONS

RADIOMETRIC ASSAY-TYPE 1
WELLS COATED WITH 60µg/ml SOLUTION OF $YD_4K$-BSA AND TESTED WITH $Ca^{2+}$-INDEPENDENT
ANTIBODY AT WORKING DILUTION 1:50. DISPLACEMENT WITH $D_4K$. DETECTION WITH $^{125}$I-GOAT
ANTI-RABBIT.

LOG MOLARITY($D_4K$)

Fig. 6

EP 0 258 995 B1

RADIOMETRIC ASSAY - TYPE 2

WELLS COATED WITH 50 μg/ml SOLUTION OF $Ca^{2+}$-INDEPENDENT ANTIBODIES. COMPETITIVE DISPLACEMENT OF $^{125}I$-YD$_4$K WITH D$_4$K

CPM

LOG MOLARITY (D$_4$K)

Fig. 7

EP 0 258 995 B1

GENERATION OF D$_4$KFROMDOG PANCREATIC SLICES BY
ENTEROKINASE ACTIVATION OF TRYPSINOGEN

o + ENTEROKINASE
□ no ENTEROKINASE

Fig.8

HOURS

EP 0 258 995 B1

CLEARANCE FROM SERUM OF A SINGLE INTRAVENOUS
DOSE OF $YD_4K$ IN A 30 kg. GREYHOUND

o SERUM
□ URINE

Fig. 9

EP 0 258 995 B1

ELUTION PROFILE AND (D₄K) OF SERUM OF J.S. ON G₁₅ CHROMATOGRAPHY.

Fig. 10

TAP ASSAY PERFORMED BLIND ON 279 STORED PANCREATITIC SERA

SAMPLES ON ADMISSION AND AT 9a.m. FOR 1-5 DAYS FROM 69 PATIENTS
AT GLASGOW ROYAL INFIRMARY

| MILD<br>BY:<br><br>RANSON/IMRIE/CLINICAL | MODERATE/SERVERE<br>BY 1 OR MORE OF:<br><br>RANSON/IMRIE/CLINICAL |
|---|---|
| 41 PATIENTS | 28 PATIENTS |

SERUM TAP ASSAY

| − | + | + | − |
|---|---|---|---|
| 36 | 2 | 3 | 13 | 5 | 10 |

% CORRECT/INCORRECT  88%  |12%|  46%  |  54%

OVERALL 71%

ADJUSTED FOR   RECURRENT/CHRONIC 2          5     2 HYPOTHERMIA
                                                  1 RTA
                                                  2 SINGLE SAMPLES

| − | + | + | − |
|---|---|---|---|
| 36 | 3 | 13 | 10 |

92%   |8%|  56%   44%

OVERALL 79%

Fig. 11

EP 0 258 995 B1

SERIAL SERUM AND URINE LEVELS OF $D_4K$ IN 3 PATIENTS
ADMITTED WITH ACUTE PANCREATITIS

Fig.12